(19) Europäisches Patentamt / European Patent Office / Office européen des brevets

(11) EP 2 988 722 B1

(12) **EUROPEAN PATENT SPECIFICATION**

(45) Date of publication and mention of the grant of the patent:
**05.12.2018 Bulletin 2018/49**

(21) Application number: **14719779.2**

(22) Date of filing: **28.04.2014**

(51) Int Cl.:
***A61K 8/9789*** *(2017.01)* ***A61K 8/34*** *(2006.01)*
***A61K 8/60*** *(2006.01)* ***A61Q 5/06*** *(2006.01)*
***A61K 8/92*** *(2006.01)* ***A61K 36/185*** *(2006.01)*

(86) International application number:
**PCT/EP2014/058591**

(87) International publication number:
**WO 2014/174113 (30.10.2014 Gazette 2014/44)**

(54) **COMPOSITION COMPRISING HENNA AND/OR INDIGO, AN OIL AND A SACCHARIDE, AND HAIR DYEING PROCESS USING THE SAME**

ZUSAMMENSETZUNG MIT HENNA UND/ODER INDIGO, EINEM ÖL UND EINEM SACCHARID SOWIE HAARFÄRBEVERFAHREN DAMIT

COMPOSITION COMPRENANT DU HENNÉ ET/OU DE L'INDIGO, UNE HUILE ET UN SACCHARIDE, ET PROCÉDÉ DE COLORATION DES CHEVEUX L'UTILISANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.04.2013 FR 1353851**

(43) Date of publication of application:
**02.03.2016 Bulletin 2016/09**

(73) Proprietor: **L'OREAL**
**75008 Paris (FR)**

(72) Inventors:
- **RHARBI, Samira**
  **F-93800 Epinay sur Seine (FR)**
- **PERON, Marine**
  **F-75010 Paris (FR)**

(74) Representative: **Rivière, François Armand**
**L'Oréal**
**Service DIPI**
**9 Rue Pierre Dreyfus**
**92110 Clichy (FR)**

(56) References cited:
**EP-A2- 0 806 199** **EP-A2- 0 806 200**
**DE-U1- 20 100 721**

- **"Tout sur le Henné et la Coloration Végétale", INTERNET CITATION, 27 September 2012 (2012-09-27), XP002720739, Retrieved from the Internet: URL:http://byreo.canalblog.com/archives/2012/09/27/24643864.html [retrieved on 2014-02-19]**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 988 722 B1

## Description

The invention relates to i) a composition comprising a) at least 10% by weight of henna and/or indigo-producing plant powder, b) at least one oil, c) at least one saccharide, with a b)/c) weight ratio > 1; ii) a process for dyeing keratin fibres, especially human keratin fibres such as the hair using the said composition; and iii) the use for dyeing keratin fibres of the combination a), b and c) as defined previously, with a b)/c) weight ratio > 1.

Two major methods for dyeing human keratin fibres, and in particular the hair, are known.

The first, known as oxidation dyeing or permanent dyeing, consists in using one or more oxidation dye precursors, more particularly one or more oxidation bases optionally combined with one or more couplers.

Oxidation bases are usually selected from ortho- or para-phenylenediamines, ortho- or para-aminophenols, and heterocyclic compounds. These oxidation bases are colourless or weakly coloured compounds, which, when combined with oxidizing products, can give rise via a process of oxidative condensation to coloured species, which remain trapped within the fibre.

The shades obtained with these oxidation bases are often varied by combining them with one or more couplers, these couplers being chosen especially from aromatic meta-diamines, meta-aminophenols, meta-diphenols and certain heterocyclic compounds, such as indole compounds.

The variety of molecules used as oxidation bases and couplers allows a wide range of colours to be obtained.

The second dyeing method, known as direct dyeing or semi-permanent dyeing, comprises the application of direct dyes, which are coloured and colouring molecules that have affinity for fibres. Given the nature of the molecules used, they tend rather to remain on the surface of the fibre and penetrate relatively little into the fibre, when compared with the small molecules of oxidation dye precursors. The main advantages of this type of dyeing are that it does not require any oxidizing agent, which limits the degradation of the fibres, and that it does not use any dyes that have particular reactivity, resulting in limitation of the intolerance risks.

The first hair dyes were semi-permanent. One of the most well known natural dyes is that derived from the henna plant. Henna continues to be used in feminine beauty enhancement for colouring the hair or the nails, or for dyeing leather, silk and wool, etc. It is also used traditionally for various important events, celebrations and beliefs.

Red henna consists of leaves of shrubs of the genus *Lawsonia* from the family of *Lythraceae*, which is based on the principle of dyeing with the active agent lawsone: 2-hydroxy-1,4-naphthoquinone. Lawsone [83-72-7] (CI Natural Orange 6; CI 75420), also known as isojuglone, may be found in henna shrubs (*Lawsonia alba, Lawsonia inermis*) ("Dyes, Natural", *Kirk-Othmer Encyclopedia of Chemical Technology, "Henna" Encyclopedia Britannica*).

This dye affords an orange-red coloration on grey hair, and a "warm" i.e. coppery to red colour on chestnut-brown hair. The dyeing process using henna is difficult to perform. A kind of "paste" (often referred to as a "poultice") is first made from ground or powdered henna leaves, which is then diluted at the time of use with warm water, and the said paste is then applied to the keratin fibres.

However, this process using the said paste has drawbacks. During the preparation and application of the composition to keratin fibres, it is not always possible to obtain satisfactory impregnation due to the poor consistency of the composition obtained from the coarsely ground powder. Furthermore, it is very difficult to hope to reproduce the shades exactly, since the lawsone content very often varies from one batch to another and between different ground materials.

Another very well-known natural dye is indigo (see Ullmann's Encyclopedia of Industrial Chemistry, Hair preparation, point 5.2.3, 2006 Wiley-VCH Verlag GmbH & Co. KGaA, Weinheim; 10.1002/14356007.a12 571.pub2). Indigo continues to be used for feminine beauty enhancement by dyeing the hair or the nails, or for dyeing fabrics (jeans), leather, silk, wool, etc. Indigo [482-89-3] is a natural dye, originating especially from the indigo plant, and having the empirical formula: $C_{16}H_{10}N_2O_2$; and having the structure:

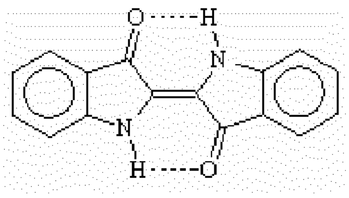

Indigo is derived from indican and may be prepared from various plants known as indigo-producing plants such as *Indigofera tinctoria*, *Indigo suffruticosa*, *Isatis tinctoria,* etc. (see Kirk-Othmer Encyclopedia of Chemical Technology, updated on 17/04/2009, DOI: 10.1002/0471238961.0425051903150618.a01.pub2). The indigo-producing plants are generally chopped and soaked in hot water, heated, fermented and oxidized in the open air to liberate the purple-blue coloured indigo (see Chem. Rev. 2011, 111, 2537-2561, pp. 2537-2561). Indigo is the result of the fermentation and

then oxidization of indican (glycosyl precursor). The indigo molecule is insoluble in water.

The problem is that dyeing using the indigo leaf is difficult because the uptake of the colour into the keratin fibres is very poor. This dye affords a blue coloration on grey hair, and a "cold" colour of ash to violet colour on chestnut-brown hair. The dyeing process using indigo leaves is difficult to perform. A kind of "paste" (often referred to as a poultice) is first made from ground or powdered leaves of indigo plant (or Indian indigo or dyer's indigo) or dyer's pastel (or woad or *Isatis tinctoria*), which needs to have been fermented, and which is then diluted at the time of use with warm water, and the said paste is then applied to the keratin fibres. However, this process using the said paste has drawbacks. During the preparation and application of the composition to keratin fibres, it is not always possible to obtain satisfactory impregnation due to the poor consistency of the composition obtained from the coarsely ground powder. Furthermore, it is very difficult to hope to reproduce the shades exactly, since the indigo content very often varies from one batch to another and between different ground materials.

As much as the colour obtained on chestnut-brown hair has a natural look, grey hair is equally dyed an unaesthetic and unnatural orange colour with henna or blue colour with indigo ("Hair preparations", Kirk-Othmer Encyclopedia of Chemical Technology, John Wiley & Sons, Inc.). Furthermore, the colorations obtained are not uniform between the root and the end or from one fibre to another (The Science of Hair Care, C. Bouillon, J. Wilkinson, 2d Ed., CRC Press, Taylor & Francis Group; Boca Raton, London, pp. 236-241 (2005)).

Added to this are the risks of staining of clothing and the skin with henna or indigo during the preparation of the "paste" and also during its application to the keratin fibres, since the consistency is very irregular.

The current colorations with henna products and products derived from indigo-producing plant(s) are applied in the form of a poultice for a long leave-on time on the hair and are then rinsed out, and the hair is dried, generally in the open air without final shampooing, so as to allow the coloration to become oxidized in the course of the following hours or even days. Depending on the composition used, the oxidation time is more or less long, but is never instantaneous.

Besides the leave-on time, which is long with henna or indigo and which may range from a few tens of minutes to a few hours (overnight) depending on the desired intensity, without being able to control the result, the result also varies as a function of the fibres to be dyed and of the indigo or henna raw material used.

It is known practice to use metal salts as mordants for improving the coloration of henna and indigo (Ullmann's Encyclopedia, 2006 Wiley-VCH Verlag GmbH & Co. KGaA, Weinheim 10.1002/14356007.a12 571.pub2 and US 2010/03133362). It is also known practice to use metal salts to improve the dyeing of indigo (vat dyes - see Chem. Rev. 2011, 111, 2537-2561, pp. 2537-2561). The use of these agents requires great know-how, multiplies the steps of the process, is not always friendly towards the integrity of the fibre (cosmetically unfriendly) and may disrupt subsequent cosmetic treatments.

It is also known practice to use a heating iron combined with a non-natural direct dye for dyeing the hair (EP 1 631 241). However, the colorations are not always satisfactory from the point of view of the colour, especially in terms of selectivity from the root to the end and of persistence, and of the cosmetic aspect of the fibres.

To overcome the problem of the poor dyeing efficacy of natural dyes, it is moreover known practice to "*dope*" the coloration by adding direct dyes that are generally used in direct dyeing, such as nitrobenzene, anthraquinone, nitropyridine, azo, methine, azomethine, xanthene, acridine, azine or triarylmethane direct dyes (DE 199 05 707, EP 0 806 199, JP 2010-0001278). This option has the drawback for natural product users, or for partisans of *"natural/bio"* products, in that the coloration is partly performed using synthetic dyes.

There is thus a real need to develop dyeing processes using natural dyes that can produce powerful colorations using henna and/or indigo-producing plant(s), while at the same time respecting the cosmetic aspect of keratin fibres. In particular, there is a need to provide keratin fibre dyeing processes using natural dyes, that are quick and easy to use and can especially produce colorations that are less aggressive to the hair and at the same time that are resistant to external agents (light, bad weather or shampooing), and that are fast and homogeneous, while at the same time remaining powerful, intensive and/or chromatic.

This/these aim(s) are achieved with the present invention, a first subject of which is a cosmetic composition A comprising:

a) at least 10% by weight of henna and/or indigo-producing plant powder;
b) at least one oil selected from jojoba oil, babassu oil, sunflower oil, olive oil, coconut oil, Brazil nut oil, marula oil, corn oil, argan oil, soybean oil, marrow oil, grapeseed oil, linseed oil, sesame oil, hazelnut oil, apricot oil, macadamia oil, arara oil, coriander oil, almond oil, avocado oil, shea butter oil, rapeseed oil, borage oil, evening primrose oil, pomegranate oil, mango oil, palm oil, cottonseed oil and copra oil ;
c) at least one optionally reduced saccharide;

it being understood that:

- the b)/c) weight ratio is greater than 1, i.e. the weight amount of oil(s) is greater than the weight amount of sugar; and
- when the composition contains indigo-producing plant powder, the saccharide is other than glucose.

The cosmetic composition A is preferably in compact and/or anhydrous form.

Another subject of the invention is the aqueous composition: (composition B) derived from the mixture between composition A as defined previously, which is preferably compact and/or anhydrous, with an aqueous composition and more preferentially water.

A subject of the invention is also a process for dyeing keratin fibres, especially human keratin fibres such as the hair, using composition A or B as defined previously.

Furthermore, a subject of the invention is the use of compositions A and B as defined previously for dyeing keratin fibres, especially human keratin fibres such as the hair.

Compositions **A** and **B** according to the invention have the advantage of dyeing keratin fibres, especially human keratin fibres, with strong, chromatic dyeing results that are resistant to washing, perspiration, sebum and light, and that are moreover long-lasting, without impairing the said fibres. Furthermore, the colorations obtained give uniform colours from the root to the end of a fibre (little coloration selectivity). Furthermore, the use of the compositions according to the invention does not give off any raw material dust (dust-free). Compositions **A** and **B** or the poultice are easy to use, in total safety and with no risk of staining. In addition, the composition and the active agent remain stable on storage. The treated keratin fibres have a very pleasant cosmetic aspect, their integrity is respected.

In addition, the composition of the invention, even in compact form, is very water-miscible even in cold water (especially between 10°C and room temperature, 25°C), and composition **B** leads to a poultice that is particularly creamy and/or shows excellent adhesion to the hair. Moreover, the time and/or ease of breakdown of the composition when it is in compact and preferably anhydrous form is faster or easier, for an equivalent amount, than the compact compositions on the market.

Moreover, the time devoted to dyeing using a natural product according to the process of the invention is shorter and the process is easier, without the need to keep a composition such as a poultice on the head for a long time (for several hours or even overnight) or to leave the composition (for example the poultice) to stand before application for a long time (several hours or several days).

*a) henna and/or indigo-producing plant(s) in powder form*

Composition A or B comprises a) henna and/or indigo-producing plant powder. It is understood that the henna and/or indigo-producing plant powder is different from an extract. Specifically, an extract is a product of maceration in solvents, generally organic solvents, whereas the powder according to the invention is a pure natural product originating from henna or indigo-producing plants, reduced by grinding or other mechanical means, into fine particles.

According to a particular embodiment of the invention, the composition used in the keratin fibre dyeing process comprises as sole ingredient a) at least 10% by weight of henna in powder form, preferably as fine particles, relative to the total weight of the said composition. The henna used in the invention is preferably red henna (*Lawsonia inermis, alba*).

The henna powder may be screened to obtain particles with upper limit sizes corresponding to the orifices or mesh sizes of the screen particularly between 35 and 80 mesh (US).

According to one particular mode of the invention, the size of the henna powder particles is fine. According to the invention, a particle size of less than or equal to 500 μm is more particularly intended. Preferentially, the powder consists of fine particles with sizes inclusively between 50 and 300 μm and more particularly between 10 and 200 μm.

It is understood that the said henna particles preferentially have a moisture content of between 0 and 10% by weight, relative to the total weight of the powders.

Preferably, the said henna particles are derived from henna leaves. Composition **B** according to the invention comprises henna powder in an amount particularly inclusively between 10% and 90% by weight, more particularly between 15% and 70%, or even between 20% and 60% by weight and more particularly between 25% and 50% by weight, relative to the total weight of the said composition.

Composition **A** comprises henna powder in an amount particularly inclusively between 15% and 100% by weight, more particularly between 15% and 80%, or even between 20% and 70% by weight and more particularly between 25% and 60% by weight, relative to the total weight of the said composition.

According to another particular embodiment of the invention, composition **A** or **B** used in the keratin fibre dyeing process comprises at least 10% by weight of powder of indigo-producing plant(s), preferably as fine particles, relative to the total weight of the composition.

As indigo-producing plants, mention may be made of numerous species derived from the following genera:

- *Indigofera* such as *Indigofera tinctoria, Indigo suffruticosa, Indigofera articulata, Indigofera arrecta, Indigofera gerardiana, Indigofera argenta, Indigofera indica, Indigofera longiracemosa;*

- *Isatis* such as *Isatis tinctoria;*
- *Polygonum* or *Persicaria* such as *Polygonum tinctorum (Persicaria tinctoria);*
- *Wrightia* such as *Wrightia tinctoria;*
- *Calanthe* such as *Calanthe veratrifolia;* and
- *Baphicacanthus* such as *Baphicacanthus cusia.*

Preferably, the indigo-producing plant is of the genus *Indigofera* and more particularly is *Indigofera tinctoria.*

Use may be made of all or part (in particular the leaves especially for *Indigofera tinctoria*) of the indigo-producing plant.

The indigo-producing plant powder may be screened to obtain particles with upper limit sizes corresponding to the orifices or mesh sizes of the screen particularly between 35 and 80 mesh (US).

According to a particular mode of the invention, the size of the indigo-producing plant powder particles is fine. According to the invention, a particle size of less than or equal to 500 μm is more particularly intended. Preferentially, the powder consists of fine particles inclusively between 50 and 300 μm and more particularly between 10 and 200 μm in size.

It is understood that the said indigo-producing plant particles preferentially have a moisture content of between 0 and 10% by weight relative to the total weight of the powders.

Composition **B** according to the invention comprises indigo-producing plant powder in an amount particularly inclusively between 10% and 90% by weight, more particularly between 15% and 70%, or even between 20% and 60% by weight and more particularly between 25% and 50% by weight, relative to the total weight of the said composition. Composition **A** comprises indigo-producing plant powder in an amount particularly inclusively between 15% and 100% by weight, more particularly between 20% and 80%, or even between 20% and 70% by weight and more particularly between 25% and 60% by weight, relative to the total weight of the said composition.

According to another advantageous embodiment of the invention, composition A or B used in the invention comprises a mixture of red henna powder as defined previously and of indigo-producing plant powder, preferably as fine particles, as defined previously.

The weight ratio of henna powder and of indigo-producing plant powder preferably ranges from 95% to 5% of henna powder per 5% to 95% of indigo-producing plant powder, particularly from 75% to 25% of henna powder per 25% to 75% of indigo-producing plant powder, and more particularly from 55% to 45% of henna powder per 45% to 55% of indigo-producing plant powder. A mixture of 50% henna powder and 50% indigo-producing plant powder is particularly estimated.

Composition **B** according to the invention comprises a mixture of henna powder + indigo-producing plant powder in an amount particularly inclusively between 10% and 90% by weight, more particularly between 15% and 70%, or even between 20% and 60% by weight and more particularly between 25% and 50% by weight, relative to the total weight of the said composition.

Composition **A** comprises a mixture of henna powder + indigo-producing plant powder in an amount particularly inclusively between 15% and 100% by weight, more particularly between 20% and 80%, or even between 25% and 70% by weight and more particularly between 25% and 60% by weight, relative to the total weight of the said composition.

*ii) The oil(s)*

Composition **A** or **B** of the invention also comprises b) one or more identical or different oils.

The term "*oil*" means a *"fatty substance"* that is liquid at room temperature (25°C) and at atmospheric pressure (760 mmHg); the viscosity at 25°C is preferably less than 1200 cps and better still less than 500 cps (defined, for example, from the Newtonian plateau determined using an ARG2 rheometer from TA Instruments equipped with a spindle with cone-plate geometry 60 mm in diameter and with an angle of 2 degrees over a shear stress range of from 0.1 Pa to 100 Pa).

The term *"fatty substance"* means an organic compound that is insoluble in water at ordinary temperature (25°C) and at atmospheric pressure (760 mmHg) (solubility of less than 5%, preferably less than 1% and even more preferentially less than 0.1%). They have in their structure at least one hydrocarbon-based chain comprising at least 6 carbon atoms or a sequence of at least two siloxane groups. In addition, the fatty substances are generally soluble in organic solvents under the same temperature and pressure conditions, for instance chloroform, dichloromethane, carbon tetrachloride, ethanol, benzene, toluene, tetrahydrofuran (THF), liquid petroleum jelly or decamethylcyclopentasiloxane. The oils are chosen from oils of natural origin, more particularly oils of plant origin, preferentially chosen from jojoba oil, babassu oil, sunflower oil, olive oil, coconut oil, Brazil nut oil, marula oil, corn oil, argan oil, soybean oil, marrow oil, grapeseed oil, linseed oil, sesame oil, hazelnut oil, apricot oil, macadamia oil, arara oil, coriander oil, almond oil, avocado oil, shea butter oil, rapeseed oil, borage oil, evening primrose oil, pomegranate oil, mango oil, palm oil, cottonseed oil and copra oil. More particularly, the oils of plant origin are chosen from avocado oil, olive oil, coconut oil, copra oil, argan oil and sunflower oil; preferentially, the oil(s), ingredient b) of the invention, are chosen from copra oils.

According to another preferred embodiment the oils of the invention is olive oil.

Composition **B** used in the process of the invention preferably comprises one or more oils in an amount particularly inclusively between 1% and 80% by weight, more particularly between 2% and 50% by weight, preferentially between 3% and 40% by weight and more preferentially between 5% and 25% by weight, relative to the total weight of the said composition.

Composition **A** may comprise one or more oils in an amount particularly inclusively between 1% and 80% by weight, relative to the total weight of the composition, more particularly between 5% and 60% by weight, preferentially between 10% and 40% by weight and more preferentially between 15% and 30% by weight, relative to the total weight of the said composition.

*Fatty substances other than oils: butters, waxes or resins*

According to another particular embodiment of the invention, composition **A** or **B** used in the process of the invention comprises one or more identical or different butters.

For the purposes of the present invention, the term *"butter"* (also known as a "pasty fatty substance") means a lipophilic fatty compound with a reversible solid/liquid change of state, comprising at a temperature of 25°C and at atmospheric pressure (760 mmHg), a liquid fraction and a solid fraction.

In other words, the starting melting point of the pasty compound can be less than 25°C. The liquid fraction of the pasty compound measured at 25°C can represent 9% to 97% by weight of the compound. This liquid fraction at 25°C preferably represents between 15% and 85% and more preferably between 40% and 85% by weight.

Preferably, the butter(s) have an end melting point of less than 60°C.

Preferably, the butter(s) have a hardness of less than or equal to 6 MPa.

Preferably, the butters or pasty fatty substances have, in the solid state, an anisotropic crystal organization, which is visible by X-ray observation.

For the purposes of the invention, the melting point corresponds to the temperature of the most endothermic peak observed on thermal analysis (DSC) as described in standard ISO 11357-3; 1999. The melting point of a pasty substance or of a wax may be measured using a differential scanning calorimeter (DSC), for example the calorimeter sold under the name DSC Q2000 by the company TA Instruments.

As regards the measurement of the melting point and the determination of the end melting point, the sample preparation and measurement protocols are as follows:
A sample of 5 mg of pasty fatty substance, preheated to 80°C and withdrawn with magnetic stirring using a spatula that is also heated, is placed in a hermetic aluminium capsule, or a crucible. Two tests are performed to ensure the reproducibility of the results.

The measurements are performed on the abovementioned calorimeter. The oven is flushed with nitrogen. Cooling is performed by an RCS 90 heat exchanger. The sample is then subjected to the following protocol: it is first placed at a temperature of 20°C, and then subjected to a first temperature rise passing from 20°C to 80°C, at a heating rate of 5°C/minute, then is cooled from 80°C to -80°C at a cooling rate of 5°C/minute and finally subjected to a second temperature rise passing from -80°C to 80°C at a heating rate of 5°C/minute. During the second temperature increase, the variation of the difference in power absorbed by the empty crucible and by the crucible containing the sample of butter is measured as a function of the temperature. The melting point of the compound is the temperature value corresponding to the top of the peak of the curve representing the variation in the difference in power absorbed as a function of the temperature.

The end melting point corresponds to the temperature at which 95% of the sample has melted.

The liquid fraction by weight of the butter at 25°C is equal to the ratio of the heat of fusion consumed at 25°C to the heat of fusion of the butter.

The heat of fusion of the pasty compound is the heat consumed by the compound in order to pass from the solid state to the liquid state. The butter is said to be in the solid state when all of its mass is in crystalline solid form. The butter is said to be in the liquid state when all of its mass is in liquid form.

The heat of fusion of the butter is equal to the integral of the entire melting curve obtained using the abovementioned calorimeter, with a temperature rise of 5 or 10°C/minute, according to standard ISO 11357-3:1999. The heat of fusion of the butter is the amount of energy required to make the compound change from the solid state to the liquid state. It is expressed in J/g.

The heat of fusion consumed at 25°C is the amount of energy absorbed by the sample to change from the solid state to the state that it has at 25°C, composed of a liquid fraction and a solid fraction.

The liquid fraction of the butter measured at 32°C preferably represents from 30% to 100% by weight of the compound, preferably from 50% to 100%, more preferably from 60% to 100% by weight of the compound. When the liquid fraction of the butter measured at 32°C is equal to 100%, the temperature of the end of the melting range of the pasty compound is less than or equal to 32°C.

The liquid fraction of the butter measured at 32°C is equal to the ratio of the heat of fusion consumed at 32°C to the heat of fusion of the pasty compound. The heat of fusion consumed at 32°C is calculated in the same way as the heat of fusion consumed at 23°C.

As regards the measurement of the hardness, the sample preparation and measurement protocols are as follows:

Composition **A** according to the invention or the butter is placed in a mould 75 mm in diameter, which is filled to about 75% of its height. In order to overcome the thermal history and to control the crystallization, the mould is placed in a Vötsch VC 0018 programmable oven, where it is first placed at a temperature of 80°C for 60 minutes, then cooled from 80°C to 0°C at a cooling rate of 5°C/minute, and then left at the stabilized temperature of 0°C for 60 minutes, and then subjected to a temperature rise ranging from 0°C to 20°C, at a heating rate of 5°C/minute, and then left at the stabilized temperature of 20°C for 180 minutes.

The compression force measurement is taken using a TA/TX2i texturometer from Swantech. The spindle used is chosen according to the texture:

- cylindrical steel spindle 2 mm in diameter for very rigid starting materials;
- cylindrical steel spindle 12 mm in diameter for sparingly rigid starting materials.

The measurement comprises three steps:

- a first step after automatic detection of the surface of the sample, where the spindle moves at a measuring speed of 0.1 mm/second, and penetrates into the composition according to the invention or the butter to a penetration depth of 0.3 mm, and the software notes the maximum force value reached;
- a second step, known as relaxation, where the spindle remains in this position for one second and the force is noted after 1 second of relaxation; and finally
- a third step, known as withdrawal, where the spindle returns to its original position at a speed of 1 mm/second, and the withdrawal energy of the spindle (negative force) is noted.

The hardness value measured during the first step corresponds to the maximum compression force measured in newtons divided by the area of the texturometer cylinder expressed in $mm^2$ in contact with the butter or the composition according to the invention. The hardness value obtained is expressed in megapascals or MPa.

According to a preferred mode of the invention, the particular butter(s) are of plant origin, such as those described in Ullmann's Encyclopedia of Industrial Chemistry ("Fats and Fatty Oils", A. Thomas, published on 15/06/2000, DOI: 10.1002/14356007.a10_173, point 13.2.2.2. Shea Butter, Borneo Tallow, and Related Fats (Vegetable Butters)).

Mention may be made more particularly of shea butter, Karité Nilotica butter (*Butyrospermum parkii*), galam butter, (*Butyrospermum parkii*), Borneo butter or fat or tengkawang tallow (*Shorea stenoptera*), shorea butter, illipé butter, madhuca butter or *Bassia madhuca longifolia* butter, mowrah butter (*Madhuca latifolia*), katiau butter (*Madhuca mottleyana*), phulwara butter (*M. butyracea*), mango butter (*Mangifera indica*), murumuru butter (*Astrocaryum murumuru*), kokum butter (*Garcinia indica*), ucuuba butter (*Virola sebifera*), tucuma butter, painya butter (*Kpangnan*) (*Pentadesma butyracea*), coffee butter (*Coffea arabica*), apricot butter (*Prunus armeniaca*), macadamia butter (*Macadamia ternifolia*), grapeseed butter (*Vitis vinifera*), avocado butter (*Persea gratissima*), olive butter (*Olea europaea*), sweet almond butter (*Prunus amygdalus dulcis*), cocoa butter (*Theobroma cacao*) and sunflower butter.

According to one preferred mode of the invention, the weight content of $C_{16}$ fatty acid triglycerides, expressed relative to the total amount of fatty acid triglycerides in the butter(s) according to the invention, is less than 23%.

Preferentially, the butter(s) according to the invention are chosen from murumuru butter, ucuuba butter, shorea butter, illipé butter, shea butter and cupuacu butter, and even more preferentially from murumuru butter and ucuuba butter.

In one preferred variant of the invention, the weight content of $C_{16}$ fatty acid triglycerides, expressed relative to the total amount of fatty acid triglycerides, ranges from 0 to 22%, better still from 0 to 15% and even better still from 2% to 12%.

Composition **B** used in the process of the invention may comprise one or more oils in an amount particularly inclusively between 1% and 80% by weight, more particularly between 2% and 50% by weight, preferentially between 3% and 40% by weight and more preferentially between 5% and 25% by weight, relative to the total weight of the said composition.

Composition **A** may comprise one or more oils in an amount particularly inclusively between 1% and 80% by weight, relative to the total weight of the composition, more particularly between 5% and 60% by weight, preferentially between 10% and 40% by weight and more preferentially between 15% and 30% by weight, relative to the total weight of the said composition.

According to another preferred embodiment of the invention, composition **A** or **B** according to the invention comprises as ingredient b) a mixture of one or more identical or different oils, as defined previously, and of one or more

identical or different butters, as defined previously.

In particular, composition **B** of the invention comprises one or more oils and one or more butters in which the amount [oil(s) + butter(s)] is inclusively between 1% and 80% by weight, more particularly between 2% and 50% by weight, preferentially between 3% and 40% by weight and more preferentially between 5% and 25% by weight, relative to the total weight of the said composition.

Composition **A** may comprise one or more oils and one or more butters in which the amount [oil(s) + butter(s)] is particularly inclusively between 1% and 80% by weight, relative to the total weight of the composition, more particularly between 5% and 60% by weight, preferentially between 10% and 40% by weight and more preferentially between 15% and 30% by weight, relative to the total weight of the said composition.

According to a particular embodiment of the invention, composition **A** or **B** also comprises one or more fatty substances other than the oils and butters b) as defined previously.

According to a particular embodiment of the invention, the composition also comprises one or more waxes, preferably of plant origin.

The waxes may be fatty alcohols or fatty esters that are solid at room temperature and at atmospheric pressure.

According to one particular embodiment of the invention, the composition comprises as third constituent one or more solid fatty alcohols, preferably of plant origin.

The fatty alcohols that are suitable for use in the invention are more particularly chosen from linear saturated alcohols comprising from 6 to 30 carbon atoms and preferably from 8 to 30 carbon atoms. Mention may be made, for example, of cetyl alcohol, stearyl alcohol and a mixture thereof (cetearyl alcohol).

As regards the solid esters of fatty acids and/or of fatty alcohols, mention may preferably be made of esters of saturated linear fatty acids and of saturated linear fatty alcohols, such as cetyl palmitate, stearyl stearate and cetyl stearate.

According to another particular embodiment of the invention, the composition also comprises one or more waxes, other than the fatty alcohols and fatty esters mentioned above, preferably of plant origin.

These (non-silicone) waxes are chosen especially from carnauba wax, candelilla wax, esparto wax, paraffin wax, ozokerite, plant waxes, such as olive tree wax, rice wax, hydrogenated jojoba wax or absolute flower waxes, such as the blackcurrant blossom essential wax sold by the company Bertin (France), animal waxes, such as beeswaxes or modified beeswaxes (cerabellina); other waxes or waxy raw materials that can be used according to the invention are in particular marine waxes, such as that sold by the company Sophim under the reference M82, polyethylene waxes or polyolefin waxes in general.

According to another particular embodiment of the invention, composition **A** or **B** also comprises one or more silicone waxes, resins or gums.

In the category of polydialkylsiloxanes, mention may be made of the waxes sold under the names Abil Wax® 9800 and 9801 by the company Goldschmidt, which are polydi($C_1$-$C_{20}$)alkylsiloxanes.

The silicone gums that may be used in accordance with the invention are especially polydialkylsiloxanes and preferably polydimethylsiloxanes with high number-average molecular weights of between 200 000 and 1 000 000, used alone or as a mixture in a solvent. This solvent can be chosen from volatile silicones, polydimethylsiloxane (PDMS) oils, polyphenylmethylsiloxane (PPMS) oils, isoparaffins, polyisobutylenes, methylene chloride, pentane, dodecane and tridecane, or mixtures thereof.

Products that may be used more particularly in accordance with the invention are mixtures such as:

- the mixtures formed from a hydroxy-terminated polydimethylsiloxane or dimethiconol (CTFA) chain, and from a cyclic polydimethylsiloxane, also known as cyclomethicone (CTFA), such as the product Q2 1401 sold by the company Dow Corning;
- mixtures of a polydimethylsiloxane gum and a cyclic silicone, such as the product SF 1214 Silicone Fluid from the company General Electric; this product is an SF 30 gum corresponding to a dimethicone, having a number-average molecular weight of 500 000, dissolved in the oil SF 1202 Silicone Fluid corresponding to decamethylcyclopentasiloxane;
- mixtures of two PDMSs with different viscosities, and more particularly of a PDMS gum and of a PDMS oil, such as the product SF 1236 from the company General Electric. The product SF 1236 is a mixture of a gum SE 30 defined above, with a viscosity of 20 m2/s and of an oil SF 96 with a viscosity of $5\times10^{-6}$ $m^2/s$. This product preferably comprises 15% of gum SE 30 and 85% of an oil SF 96.

The organopolysiloxane resins that may be used in accordance with the invention are crosslinked siloxane systems containing the following units:

$$R_2SiO_{2/2},\ R_3SiO_{1/2},\ RSiO_{3/2} \text{ and } SiO_{4/2},$$

in which R represents an alkyl containing 1 to 16 carbon atoms. Among these products, the ones that are particularly

preferred are those in which R denotes a $C_1$-$C_4$ lower alkyl group, more particularly methyl.
Among these resins, mention may be made of the product sold under the name Dow Corning 593 or those sold under the names Silicone Fluid SS 4230 and SS 4267 by the company General Electric, which are silicones of dimethyl/trimethylsiloxane structure.

Mention may also be made of the trimethyl siloxysilicate type resins sold especially under the names X22-4914, X21-5034 and X21-5037 by the company Shin-Etsu.

Preferably, the fatty substance(s) do not comprise any $C_2$-$C_3$ oxyalkylene units or any glycerolated units.

Composition **A** or **B** of the invention preferably comprises a content of fatty substances other than the oil(s) and butter(s) as defined previously ranging from 0.5% to 50% and by weight, better still from 1% to 30% by weight and even better still from 1% to 20% by weight relative to the total weight of the said composition.

*c) The saccharide(s)*

Composition **A** or **B** of the invention contains c) one or more optionally reduced saccharides.

The saccharides may be chosen from monosaccharides, oligosaccharides and polysaccharides.

In particular, the saccharides or reduced saccharides of the invention are solid, i.e. they are not liquids or syrups.

*"Monosaccharides, oligosaccharides and polysaccharides"* are known to those skilled in the art (see, for example, Ullmann's Encyclopedia of Industrial Chemistry, published online on 15/04/2010, "Carbohydrates: Occurrence, Structures and Chemistry", F. W. Lichtenthaler, vol. 6, pp. 617 to 646, DOI: 10.1002/14356007.a05_079.pub2,).

According to a particular embodiment of the invention, the sugar(s) of the dye composition and of the dyeing process are chosen from monosaccharides.

More precisely, the term *"monosaccharide"* means a sugar comprising only one unit, i.e. not comprising any covalent glycoside bonds with another sugar.

Preferentially, the *"monosaccharides"* of the invention are chosen from:

i) *"aldoses"* or polyhydroxyaldehydes, preferably comprising between 4 and 6 carbon atoms, such as erythrose or threose (4 carbon atoms), ribose, arabinose, xylose or lyxose (5 carbon atoms), allose, altrose, glucose, mannose, gulose, idose, galactose and talose (6 carbon atoms);
ii) *"ketoses"* or polyhydroxy ketones preferably comprising between 4 and 6 carbon atoms, such as erythrulose (4 carbon atoms), ribulose, or xylulose (5 carbon atoms), psicose, fructose, sorbose and tagatose (6 carbon atoms); and

the reduced forms of the aldoses and ketoses as defined previously are also known as *"sugar alcohols"* or *"alditols".* They are chosen in particular from erythritol, glucitol or sorbitol, mannitol and xylitol, preferably sorbitol.

Sugar alcohols are known to those skilled in the art (see, for example, Ullmann's Encyclopedia of Industrial Chemistry, published online on 15/04/2010, "Carbohydrates: Occurrence, Structures and Chemistry", F. W. Lichtenthaler, vol. 6, chap. 7.6, pp. 637 and 638, DOI: 10.1002/14356007.a05_079.pub2; *ibid,* published online on 15/01/2012, "Sugar Alcohols", H. Schiweck et al., 2012 Wiley-VCH Verlag GmbH & Co. KGaA, Weinheim, DOI: 10.1002/14356007.a25_413.pub3

The term *"sugar alcohol"* means *"polyols"* generally obtained by reduction of aldose or ketose monosaccharides as defined previously or of complex oligosaccharides or polysaccharides as defined below in which the aldehyde or ketone group(s) of the monosaccharide units are reduced, i.e. replaced with a hydroxyl group.

Preferably, the sugars of the invention, and in particular the sugar alcohols according to the invention, are of plant origin.

It is understood that the terms "aldoses", "ketoses" and "sugar alcohols" also refer to the optical isomers thereof, the $\alpha$ and $\beta$ anomers thereof and the L (laevorotatory) and D (dextrorotatory) forms thereof.

More preferentially, the monosaccharides of the invention comprise 6 carbon atoms.

The term *"oligosaccharide"* means a sugar in which the monosaccharides as defined previously are combined together via a covalent glycoside bond to give a simple polymer comprising from 2 to 10 monosaccharide units.

In particular, the oligosaccharides are chosen from disaccharides such as sucrose, trehalose and raffinose, lactose, cellobiose and maltose; $\alpha$, $\beta$ or $\gamma$-cyclodextrins and the "sugar alcohol" reduced forms thereof such as isomaltulose, trehalulose, isomalt, maltitol and lactitol.

It is understood that the term "oligosaccharides" also refers to the optical isomers thereof, the $\alpha$ and $\beta$ anomers thereof and the L (laevorotatory) and D (dextrorotatory) forms thereof.

The term *"polysaccharides"* means oligosaccharides which comprise at least 11 monosaccharide units. Preferentially, the polysaccharides of the invention comprise between 20 and 100 000 monosaccharide units.

The polysaccharides of the invention may be chosen from those derived from the following sugars: i) glucose; ii) galactose; iii) arabinose; iv) rhamnose; v) mannose; vi) xylose; vii) fucose; viii) anhydrogalactose; ix) galacturonic acid; x) glucuronic acid; xi) mannuronic acid; xii) galactose sulfate; xiii) anhydrogalactose sulfate.

The polymers bearing sugar units of the invention may be natural or synthetic.
They may be nonionic, anionic, amphoteric or cationic.
According to a particular embodiment, the saccharides of the invention are chosen from:

- native gums such as:

  a) tree or shrub exudates, including:

  - gum arabic (branched polymer of galactose, arabinose, rhamnose and glucuronic acid);
  - ghatti gum (polymer derived from arabinose, galactose, mannose, xylose and glucuronic acid);
  - karaya gum (polymer derived from galacturonic acid, galactose, rhamnose and glucuronic acid);
  - gum tragacanth (or tragacanth) (polymer of galacturonic acid, galactose, fucose, xylose and arabinose);

  b) gums derived from algae, such as:

  - agar (polymer derived from galactose and anhydrogalactose);
  - alginates (polymers of mannuronic acid and of glucuronic acid);
  - carrageenans and furcellerans (polymers of galactose sulfate and of anhydrogalactose sulfate);

  c) gums derived from seeds or tubers, such as:

  - guar gum (polymer of mannose and galactose);
  - locust bean gum (polymer of mannose and galactose);
  - fenugreek gum (polymer of mannose and galactose);
  - tamarind gum (polymer of galactose, xylose and glucose);
  - konjac gum (polymer of glucose and mannose);

  d) microbial gums, such as:

  - xanthan gum (polymer of glucose, mannose acetate, mannose/pyruvic acid and glucuronic acid);
  - gellan gum (polymer of partially acylated glucose, rhamnose and glucuronic acid);
  - scleroglucan gum (glucose polymer);

  e) plant extracts, such as:

  - cellulose (glucose polymer); and
  - starch (glucose polymer).

These polymers may be physically or chemically modified. A physical treatment that may especially be mentioned is the temperature.
Chemical treatments that may be mentioned include esterification, etherification, amidation or oxidation reactions. These treatments can lead to polymers that may be nonionic, anionic, cationic or amphoteric.
Preferably, these chemical or physical treatments are applied to guar gums, locust bean gums, starches and celluloses.
The nonionic guar gums that may be used according to the invention may be modified with $C_1$-$C_6$ hydroxyalkyl groups.
Among the hydroxyalkyl groups that may be mentioned, for example, are hydroxymethyl, hydroxyethyl, hydroxypropyl and hydroxybutyl groups.
These guar gums are well known in the prior art and can be prepared, for example, by reacting the corresponding alkene oxides such as, for example, propylene oxides, with the guar gum so as to obtain a guar gum modified with hydroxypropyl groups.
The degree of hydroxyalkylation preferably ranges from 0.4 to 1.2, and corresponds to the number of alkylene oxide molecules consumed by the number of free hydroxyl functions present on the guar gum.
Such nonionic guar gums optionally modified with hydroxyalkyl groups are sold, for example, under the trade names Jaguar HP8, Jaguar HP60 and Jaguar HP120 by the company Rhodia Chimie.
The guar gums modified with cationic groups that may be used more particularly according to the invention are guar gums comprising trialkylammonium cationic groups. Preferably, 2% to 30% by number of the hydroxyl functions of these guar gums bear trialkylammonium cationic groups. Even more preferentially, 5% to 20% by number of the hydroxyl

functions of these guar gums are branched with trialkylammonium cationic groups.

Among these trialkylammonium groups, mention may be made most particularly of trimethylammonium and triethylammonium groups.

Even more preferentially, these groups represent from 5% to 20% by weight relative to the total weight of the modified guar gum.

According to the invention, use may be made of guar gums modified with 2,3-epoxypropyltrimethylammonium chloride.

These guar gums modified with cationic groups, are products already known per se and are, for example, described in patents US 3 589 578 and US 4 0131 307. Such products are moreover sold especially under the trade names Jaguar C13 S, Jaguar C 15 and Jaguar C 17 by the company Rhodia Chimie.

A modified locust bean gum that may be used is cationic locust bean gum containing hydroxypropyltrimonium groups, such as Catinal CLB 200 sold by the company Toho.

The botanical origin of the starch molecules used in the present invention may be cereals or tubers. Thus, the starches are chosen, for example, from corn starch, rice starch, cassava starch, barley starch, potato starch, wheat starch, sorghum starch and pea starch.

The starches may be chemically or physically modified especially by one or more of the following reactions: pregelatinization, oxidation, crosslinking, esterification, etherification, amidation, heat treatments.

More particularly, these reactions may be performed in the following manner:

- pregelatinization by splitting the starch granules (for example drying and cooking in a drying drum);
- oxidation with strong oxidizing agents, leading to the introduction of carboxyl groups into the starch molecule and to depolymerization of the starch molecule (for example by treating an aqueous starch solution with sodium hypochlorite);
- crosslinking with functional agents capable of reacting with the hydroxyl groups of the starch molecules, which will thus bond together (for example with glyceryl and/or phosphate groups);
- esterification in alkaline medium for the grafting of functional groups, especially $C_1$-$C_6$ acyl (acetyl), $C_1$-$C_6$ hydroxyalkyl (hydroxyethyl or hydroxypropyl), carboxymethyl or octenylsuccinic.

Monostarch phosphates of the type St-O-P(O)$(OX)_2$, distarch phosphates of the type St-OP(O)(OX)-O-St or even tristarch phosphates of the type St-O-P(O)$(O\text{-}St)_2$ or mixtures thereof may especially be obtained by crosslinking with phosphorus compounds, with

- St meaning starch;
- X especially denoting alkali metals (for example sodium or potassium), alkaline-earth metals (for example calcium or magnesium), ammonium salts, amine salts, for instance those of monoethanolamine, diethanolamine, triethanolamine, 3-amino-1,2-propanediol, or ammonium salts derived from basic amino acids such as lysine, arginine, sarcosine, ornithine or citrulline.

The phosphorus compounds may be, for example, sodium tripolyphosphate, sodium orthophosphate, phosphorus oxychloride or sodium trimetaphosphate.

Distarch phosphates or compounds rich in distarch phosphate will preferentially be used, for instance the product sold under the references Prejel VA-70-T AGGL (gelatinized hydroxypropyl cassava distarch phosphate), Prejel TK1 (gelatinized cassava distarch phosphate) and Prejel 200 (gelatinized acetyl cassava distarch phosphate) by the company Avebe, or Structure Zea from National Starch (gelatinized corn distarch phosphate).

A preferred starch is a starch that has undergone at least one chemical modification such as at least one esterification.

According to a particular mode of the invention, amphoteric starches are used as saccharides. These amphoteric starches comprise one or more anionic groups and one or more cationic groups. The anionic and cationic groups may be linked to the same reactive site of the starch molecule or to different reactive sites; they are preferably linked to the same reactive site. The anionic groups may be of carboxylic, phosphate or sulfate type, preferably carboxylic. The cationic groups may be of primary, secondary, tertiary or quaternary amine type.

The amphoteric starches are especially chosen from the compounds of formulae (A), (B), (C) and (D) below:

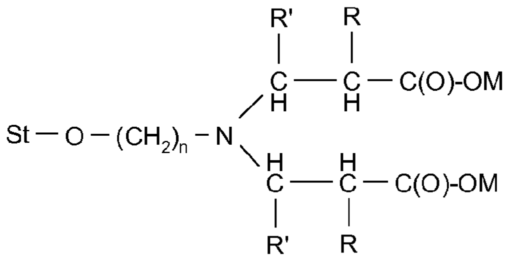

(A)

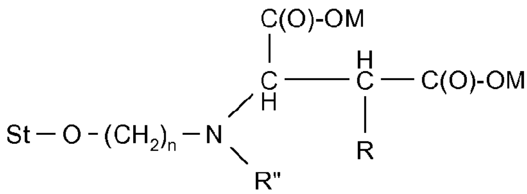

(B)

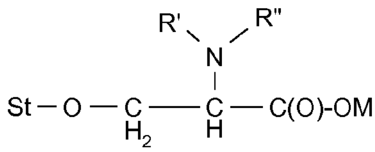

(C)

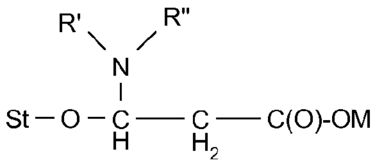

(D)

in which formulae (A), (B), (C) and (D):

- St-O represents a starch molecule;
- R, which may be identical or different, represents a hydrogen atom or a methyl radical;
- R', which may be identical or different, represents a hydrogen atom, a methyl radical or a carboxyl -C(O)-OH group;
- n is an integer equal to 2 or 3;
- M, which may be identical or different, denotes a hydrogen atom, an alkali metal or alkaline-earth metal such as $Na^+$, $K^+$, $Li^+$ or $NH_4^+$, a quaternary ammonium or an organic amine;
- R" represents a hydrogen atom or an alkyl radical containing from 1 to 18 carbon atoms.

The compounds of formulae (A) to (D) may be substituted with one or more halogen atoms.

These compounds are especially described in patents US 5 455 340 and US 4 017 460, which are included by way of reference.

The starch molecules may be derived from any plant source of starch, especially such as corn, potato, oat, rice, tapioca, sorghum, barley or wheat. It is also possible to use the starch hydrolysates mentioned above. The starch is preferably derived from potato.

The starches of formula (B) or (C) are particularly used. Starches modified with 2-chloroethylaminodipropionic acid are more particularly used, i.e. starches of formula (B) or (C) in which R, R', R" and M represent a hydrogen atom and n is equal to 2. The preferred amphoteric starch is a starch chloroethylamidodipropionate.

As mentioned previously, the cellulose derivatives may be anionic, cationic, amphoteric or nonionic.

Among these derivatives, cellulose ethers, cellulose esters and cellulose ester ethers are distinguished.

Among the cellulose esters are mineral esters of cellulose (cellulose nitrates, sulfates, phosphates, etc.), organic cellulose esters (cellulose monoacetates, triacetates, amidopropionates, acetatebutyrates, acetatepropionates and acetatetrimellitates, etc.), and mixed organic/mineral esters of cellulose, such as cellulose acetatebutyrate sulfates and cellulose acetatepropionate sulfates. Among the cellulose ester ethers, mention may be made of hydroxypropylmethylcellulose phthalates and ethylcellulose sulfates.

Among the nonionic cellulose ethers that may be mentioned are alkylcelluloses such as methylcelluloses and ethylcelluloses (for example Ethocel Standard 100 Premium from Dow Chemical); hydroxyalkylcelluloses such as hydroxymethylcelluloses and hydroxyethylcelluloses (for example Natrosol 250 HHR sold by Aqualon) and hydroxypropylcelluloses (for example Klucel EF from Aqualon); mixed hydroxyalkyl-alkylcelluloses such as hydroxypropylmethylcelluloses (for example Methocel E4M from Dow Chemical), hydroxyethylmethylcelluloses, hydroxyethylethylcelluloses (for example Bermocoll E 481 FQ from Akzo Nobel) and hydroxybutylmethylcelluloses.

Among the anionic cellulose ethers, mention may be made of carboxyalkylcelluloses and salts thereof. Examples that may be mentioned include carboxymethylcelluloses, carboxymethylmethylcelluloses (for example Blanose 7M from the company Aqualon) and carboxymethylhydroxyethylcelluloses, and the sodium salts thereof.

Among the cationic cellulose ethers, mention may be made of crosslinked or non-crosslinked, quaternized hydroxyethylcelluloses. The quaternizing agent may especially be diallyldimethylammonium chloride (for example Celquat L200 from National Starch). Another cationic cellulose ether that may be mentioned is hydroxypropyltrimethylammonium hydroxyethyl cellulose (for example Ucare Polymer JR 400 from Amerchol).

- Among the associative thickening polymers of the invention, mention may be made of celluloses or derivatives thereof, modified with groups comprising at least one fatty chain, such as alkyl, arylalkyl or alkylaryl groups or mixtures thereof in which the alkyl groups are of $C_8$-$C_{22}$;
- nonionic alkylhydroxyethylcelluloses such as the products Natrosol Plus Grade 330 CS and Polysurf 67 ($C_{16}$ alkyl) sold by the company Aqualon;
- quaternized alkylhydroxyethylcelluloses (cationic), such as the products Quatrisoft LM 200, Quatrisoft LM-X 529-18-A, Quatrisoft LM-X 529-18-B (C12 alkyl) and Quatrisoft LM-X 529-8 (C18 alkyl) sold by the company Amerchol, the products Crodacel QM and Crodacel QL (C12 alkyl) and Crodacel QS (C18 alkyl) sold by the company Croda, and the product Softcat SL 100 sold by the company Amerchol;
- nonionic nonoxynylhydroxyethylcelluloses such as the product Amercell HM-1500 sold by the company Amerchol;
- nonionic alkylcelluloses such as the product Bermocoll EHM 100 sold by the company Berol Nobel;
- Associative guar derivatives that may be used include hydroxypropyl guars modified with a fatty chain, such as the product Esaflor HM 22 (modified with a $C_{22}$ alkyl chain) sold by the company Lamberti; the product Miracare XC 95-3 (modified with a $C_{14}$ alkyl chain) and the product RE 205-146 (modified with a $C_{20}$ alkyl chain) sold by Rhodia Chimie.

Thus, more particularly, the polysaccharides of the invention are chosen from celluloses, guar gums, xanthan gums, chitins and chitosans, starches including amylose and amylopectin, dextrans and inulins.

It is understood that the term "polysaccharides" also refers to the optical isomers thereof, the $\alpha$ and $\beta$ anomers thereof and the L (laevorotatory) and D (dextrorotatory) forms thereof.

According to a particular mode of the invention, the saccharide(s) of the invention are other than sucrose.

According to a particularly advantageous mode of the invention, the saccharide(s) of the invention are chosen from monosaccharides and disaccharides and in particular from aldoses, ketoses and the reduced forms thereof, namely sugar alcohols such as sorbitol. Preferentially, sugar(s) are chosen from sugar alcohols of aldose and ketose monosaccharides such as sorbitol.

According to a particular embodiment of the invention, the saccharide(s) or the reduced forms thereof, ingredient c), are in an amount inclusively between 0.2% and 50% by weight, relative to the total weight of composition **A**, more particularly between 2% and 30% by weight, preferentially between 3% and 20% by weight and more preferentially between 5% and 15% by weight relative to the total weight of the said composition.

The saccharide(s) or the reduced forms thereof, ingredient c), are in an amount inclusively between 0.1% and 30% by weight, relative to the total weight of composition **B**, more particularly between 0.5% and 25% by weight, preferentially between 1% and 20% by weight and more preferentially between 2% and 15% by weight relative to the total weight of the said composition.

The b)/c) weight ratio is greater than 1, i.e. the weight amount of oil(s) is greater than the weight amount of saccharide(s). Preferably, this ratio ranges from 1.05 to 100 and better still from 1.1 to 10.

*Form of the compositions*

According to a preferred embodiment of the invention, composition **A** is in compact form and in different forms as a function of the desired compacting, especially in the form of pebbles, in the form of stones, in the form of soaps, in the form of pyramids, in the form of cartons or in the form of plates.

The cosmetic composition **A** of the invention may also be in non-compact galenical forms, such as a lotion, a mousse, a cream or a gel, or in any other form that is suitable for dyeing keratin fibres. It may also be conditioned in a propellant-free pump-action bottle or under pressure in an aerosol container in the presence of a propellant and form a

foam.

*The compositions*

Compositions **A** and **B** used in the process of the invention are cosmetic, i.e. they are cosmetically acceptable and therefore suitable for use for application to keratin fibres, especially human keratin fibres such as the hair.

According to a particular embodiment of the invention, composition i) used in the process of the invention contains one or more "mordants", *i.e.* metal salts conventionally used in "mordanting" (see for example Ullmann's Encyclopedia of Industrial Chemistry ("Textile Dyeing", Herbert Leube et al., DOI: 10.1002/14356007.a26_351, and in particular point 4.8.2, p. 72 ; *ibid,* "Metal-complex dyes", Klaus Gryschtol et al., DOI: 10.1002/14356007.a16_299).

According to another particular embodiment of the invention, composition i) used in the process of the invention does not contain any "mordants". Preferentially, the dyeing process of the invention does not use any mordants.

*Aqueous composition B*

Composition **B** of the invention as defined previously comprises water. This water constitutes part or all of an aqueous phase C. The term *"aqueous phase"* means a phase which comprises essentially water, and also comprises other ingredients that are water-miscible or water-soluble at room temperature and at atmospheric pressure. As liquids or solids that may be present in the aqueous phase, mention may be made of: polar or polar protic organic solvents as defined below, salts of mineral or organic acids or bases, or water-soluble cosmetic active agents.

Composition **B** of the invention contains water preferably in an amount ranging from 5% to 90%, better still from 20% to 80% and particularly from 40% to 75% by weight relative to the total weight of the said composition.

As mentioned previously, another subject of the invention is composition **B** derived from the mixture between composition **A,** which is preferably compact and/or anhydrous, and an aqueous composition **C** and preferably water (only water). It is thus possible to use in this composition **B** henna and/or indigo a) as defined previously and optionally other natural dyes as defined below, combined with the oil(s) b) as defined previously, the saccharide(s) c) as defined previously and optionally one or more fatty substances other than oils.

Preferably, composition **B** is the form of a poultice.

To do this, composition **A** according to the invention, preferably in compact and/or anhydrous form comprising the ingredients a), b) and c) as defined previously, is mixed with an aqueous composition, and preferentially mixed with water to obtain a poultice in order to obtain a creamy and pleasant consistency. When the composition is compact, it is crumbled into the aqueous composition **C,** and the compact composition is preferentially crumbled into water. The ratios of composition **A** according to the invention and an aqueous composition **C** and preferentially water preferably range from 1 part by weight of composition per 1 part by weight of aqueous composition **C** and preferentially water (1/1) to 1 part by weight of composition **A** per 3 parts by weight of aqueous composition and preferentially water (1/3), more preferentially 1 part by weight of composition **A** per 2 parts of aqueous composition **C** and preferentially water (1/2).

According to another particular embodiment of the invention, composition **B** comprises only ingredients of natural origin.

During the preparation of the poultice, one or more identical or different clays, as defined below, may be added.

According to another preferred embodiment of the invention, composition **B** is at a neutral pH close to 7 (preferably ranging from 6 to 8 and better still from 6.5 to 7.5).

As mentioned previously, composition **B,** i.e. used in the process of the invention in the first step b), may be derived from the mixture of a composition **A** mixed with water or an aqueous composition, preferably to form a poultice.

*Composition **A**:*

Composition **A** may be compacted or in the form of a non-compacted powder, and may be anhydrous or non-anhydrous.

Composition **A** may comprise water or a mixture of water and also of one or more organic solvents or a mixture of organic solvents.

Composition **A** used preferably comprises less than 3% by weight and preferably less than 2% by weight of water relative to the total weight of the composition, or even is free of water.

Preferably, composition **A** is anhydrous, i.e. it does not comprise any water other than the water associated with the starting materials included in its composition.

Compositions **A** may be in the form of a powder.

It may also be in the form of a paste.

Composition **A** according to the invention may be in compact form. As emerges from the foregoing, the compact composition according to the invention is *"solid".*

- *"solid"* means the state of the composition at room temperature (25°C) and at atmospheric pressure (760 mmHg), i.e. a composition of high consistency, which conserves its form during storage. As opposed to "fluid" compositions, it does not flow under its own weight. It is advantageously characterized by a hardness as defined below.
- *"compact composition"* means that the composition consists of a mixture of products whose cohesion is at least partly provided by compacting or pressing during the manufacture. In particular, by carrying out a measurement using a TA.XT.plus Texture Analyser sold by the company Stable Micro Systems, the compact powder according to the invention can advantageously exhibit a resistance to pressure of between 0.2 and 2.5 kg and in particular between 0.8 and 1.5 kg, with respect to the surface area of the spindle used (in the case in point, 7.07 $mm^2$). The measurement of this resistance is performed by moving an SMS P/3 flat-ended cylindrical spindle in contact with the powder over a distance of 1.5 mm and at a speed of 0.5 mm/sec.

According to a preferred embodiment of the invention, composition **A** is in compact form and in different forms as a function of the desired compacting, especially in the form of pebbles, in the form of stones, in the form of soaps, in the form of pyramids, in the form of cartons or in the form of plates. More preferentially, when the composition comprises b) one or more oils and/or butters and/or fatty substances, it is in compact form.
The cosmetic composition **A** of the invention may also be in other non-compact galenical forms, such as a lotion, a mousse, a cream, a gel, or in any other form that is suitable for dyeing keratin fibres. It may also be conditioned in a propellant-free pump-action bottle or under pressure in an aerosol container in the presence of a propellant and form a foam.
Preferably, composition **B** is the form of a *"poultice"*.
As mentioned previously, composition **B** is derived from the mixture between composition **A,** which is preferably compact and/or anhydrous, and an aqueous composition **C** and preferably water (only water) to give a mixture preferably in the form of a poultice. Use may thus be made of composition **A** comprising:

a) henna and/or indigo-producing plant powder as defined previously;
b) at least one oil and optionally at least one butter as defined previously;
c) at least one optionally reduced saccharide with a b)/c) weight ratio > 1; and
e) optionally other additional natural dyes as defined below.

To do this, composition **A** as defined previously is preferably in compact and/or anhydrous form comprising the ingredients a) to d) as defined previously is mixed with an aqueous composition, and preferentially mixed with water to obtain a poultice of creamy and pleasant consistency. When composition **A** is compact, it is crumbled into the aqueous composition **C,** and the compact composition is preferentially crumbled into water.
According to another preferred embodiment of the invention, composition **B** is at a neutral pH close to 7 (preferably ranging from 6 to 8 and better still from 6.5 to 7.5).
Compositions **A**, **B** and/or **C** may comprise one or more organic solvents. Examples of organic solvents that may be mentioned include $C_1$-$C_4$ lower alkanols, such as ethanol and isopropanol; polyols and polyol ethers such as 2-butoxyethanol, propylene glycol, propylene glycol monomethyl ether, diethylene glycol monoethyl ether and monomethyl ether, hexylene glycol, and also aromatic alcohols, for instance benzyl alcohol or phenoxyethanol.
The organic solvents are present in proportions preferably of between 0.1% and 20% by weight approximately and even more preferentially between 0.5% and 10% by weight approximately relative to the total weight of the composition under consideration.

*Adjuvants:*

Compositions **A**, **B** and/or **C** of the invention may also contain various adjuvants conventionally used in hair dye compositions other than the saccharides of the invention, such as anionic, cationic, nonionic, amphoteric or zwitterionic polymers or mixtures thereof, anionic, cationic, nonionic or zwitterionic surfactants, mineral or organic thickeners, and in particular anionic, cationic, nonionic and amphoteric polymeric associative thickeners, antioxidants, penetrants, sequestrants, fragrances, buffers, dispersants, conditioning agents other than the butters or oils of the invention, for instance ceramides, film-forming agents, preserving agents, opacifiers and mineral or organic thickeners such as clays.
According to a particular embodiment, compositions **A** and/or **B** are not in emulsion form. Preferably, in this embodiment, compositions **A** and/or **B** do not contain any surfactants.
According to another advantageous variant, compositions **A** and/or **B** are in the form of an emulsion, in which case **A** and/or **B** contain surfactants.
The above adjuvants are generally present in an amount for each of them of between 0.01% and 40% by weight relative to the weight of the composition, and preferably between 0.1% and 20% by weight relative to the weight of the composition under consideration.

Needless to say, a person skilled in the art will take care to select this or these optional additional compound(s) such that the advantageous properties intrinsically associated with the composition **A** or **B** that is useful in the dyeing process in accordance with the invention are not, or are not substantially, adversely affected by the envisaged addition(s).

*d) Additional dyes:*

Compositions **A**, **B** and/or **C** of the invention as defined previously may also contain d) one or more additional direct dyes other than the henna and/or indigo-producing plant powder a).

These direct dyes are chosen, for example, from those conventionally used in direct dyeing, and among which mention may be made of any commonly used aromatic and/or non-aromatic dyes such as neutral, acidic or cationic nitrobenzene direct dyes, neutral, acidic or cationic azo direct dyes, natural direct dyes, neutral, acidic or cationic quinone and in particular anthraquinone direct dyes, azine, triarylmethane, indoamine, methine, styryl, porphyrin, metalloporphyrin, phthalocyanine, cyanine and methine direct dyes, and fluorescent dyes.

Preferentially, compositions **A**, **B**, and/or **C** of the invention may comprise one or more natural dyes other than henna and indigo-producing plant(s) a) as defined previously. Among the natural direct dyes, mention may be made of juglone, isatin, curcumin, spinulosin, apigenidin and orceins. These natural dyes may be added in the form of defined compounds, extracts or plant parts. The said defined compounds from extracts or from plant parts are preferably in the form of powders, in particular fine powders whose particles have sizes identical to that of the henna and/or indigo-producing plant powder a) as defined previously.

The natural or non-natural direct dye(s), other than the henna and/or indigo-producing plant powder a), in the composition according to the invention particularly represents from 0.001% to 10% by weight relative to the total weight of the composition and even more preferentially from 0.05% to 5% by weight relative to the total weight of the composition under consideration.

Preferably, composition **B** of the invention does not contain any synthetic direct dyes, i.e. dyes that do not occur in nature.

Compositions **A** and **B** comprising the ingredients a) to d) as defined previously and also composition **C** according to the invention may also comprise one or more oxidation bases and/or one or more couplers conventionally used for the dyeing of keratin fibres.

Among the oxidation bases, mention may be made of para-phenylenediamines, bis(phenyl)alkylenediamines, para-aminophenols, bis-para-aminophenols, ortho-aminophenols and heterocyclic bases, and the addition salts thereof.

Preferentially, composition **B** and **A** and/or **C** of the invention does not contain any para-phenylenediamine(s).

Among these couplers, mention may be made especially of meta-phenylenediamines, meta-aminophenols, meta-diphenols, naphthalene couplers and heterocyclic couplers, and the addition salts thereof.

The oxidation base(s) present in the composition(s) are each generally present in an amount of between 0.001% and 10% by weight, relative to the total weight of the dye composition(s).

Preferably, compositions **A**, **B** and/or **C** do not contain any oxidation dyes.

*pH of compositions B and C*

According to a particular mode of the invention, the pH of the aqueous composition **B** and also the pH of the aqueous composition **C** is neutral, i.e. it has a pH of about 7 (preferably ranging from 6 to 8 and better still from 6.5 to 7.5).

According to another particular mode of the invention, composition **B** used in the process of the invention and/or composition **C** is acidic and preferably has a pH ranging from 3 to 6.5.

The pH of composition **B** and/or **C** is optionally adjusted to the desired value by means of acidifying or basifying agents usually used in the dyeing of keratin fibres or alternatively with the aid of standard buffer systems, present in composition **A** or in composition **C** mixed with composition **A** to give composition **B**.

Among the acidifying agents for the compositions used in the invention, examples that may be mentioned include mineral or organic acids, for instance hydrochloric acid, orthophosphoric acid or sulfuric acid, carboxylic acids, for instance acetic acid, tartaric acid, citric acid and lactic acid, and sulfonic acids; the acid is preferably an organic acid such as citric acid.

The alkaline agents are preferably chosen from aqueous ammonia, alkali metal carbonates or bicarbonates, alkanolamines such as monoethanolamine, diethanolamine and triethanolamine, and also derivatives thereof, sodium hydroxide, potassium hydroxide and the compounds having the following formula:

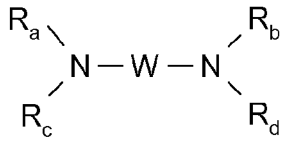

in which formula W is a linear or branched ($C_1$-$C_6$)alkylene group such as propylene, optionally substituted with a hydroxyl group or a $C_1$-$C_4$ alkyl radical; $R_a$, $R_b$, $R_c$ and $R_d$, which may be identical or different, represent a hydrogen atom or a $C_1$-$C_4$ alkyl or $C_1$-$C_4$ hydroxyalkyl radical.

*Process for preparing composition **A***

Composition **A** may be obtained in the following manner:
Ingredients a) to d) as defined are mixed together by hand or with a standard mixer and/or an extruder and/or are subjected to a standard mechanical stress pressure.

*Dyeing process using the composition of the invention*

According to one particular embodiment of the invention, the dyeing process is performed in several steps:

- the first step consists in preparing composition **B** of the invention, in particular in the form of a creamy poultice, in particular as defined previously, starting with composition **A** of the invention;
- in the second step, composition **B** is applied to the keratin fibres and is left on the said fibres preferably for a minimum time of 30 minutes, preferentially a time ranging from 30 minutes to 24 hours and better still ranging from 1 hour to 12 hours;
- in the third step, the keratin fibres are rinsed with water until composition **B** has disappeared, preferably without shampooing;
- the keratin fibres may then be dried or left to dry naturally, without a hairdryer.

According to another particular embodiment of the invention, the dyeing process is performed in several steps:

- the first step consists in preparing composition **B** of the invention as described previously;

  - in the second step, composition **B** is left to stand for several hours, preferably 24 hours, and composition **B** is then applied and left on the said fibres preferably for a minimum time of 30 minutes (preferably ranging from 30 minutes to 24 hours and better still from 1 hour to 12 hours);

- in the third step, the keratin fibres are rinsed with water until composition **B** has disappeared, preferably without shampooing;
- the keratin fibres may then be dried or left to dry naturally, without a hairdryer.

The aqueous composition mixed with composition **A**, preferably water, used in the first step may be at room temperature or at a higher temperature, in particular at a temperature ranging from 40°C to 98°C.
According to another embodiment of the invention, the composition is mixed with or crumbled into an aqueous composition, preferably water, at a temperature below 40°C, in particular between 10°C and 40°C.
Preferably, the ratio of the amount by weight of composition of the invention/amount by weight of aqueous composition and preferably water ranges from 1/1 to 1/3 and is preferably 1/2.
According to a particularly advantageous process, after the third step, the keratin fibres are:

a) either mechanically wiped with a towel or absorbent paper,
b) or dried by heat with as a heat source (convection, conduction or radiation) by passing over, for example, a stream of a warm gas such as air necessary to evaporate off the solvent(s); heat sources that may be mentioned include a hairdryer, hairdrying hoods, a hair-straightening iron, an infrared ray dispenser and any other standard heating appliances.

Irrespective of the application method, the application temperature for composition **B** ranges from room temperature (15 to 25°C) to 80°C and more particularly from 15 to 45°C. Thus, after application of composition **B** preferably in the form of a poultice according to the invention, the head of hair may advantageously be subjected to a heat treatment

by heating to a temperature ranging from 30 to 60°C. In practice, this operation may be performed using a styling hood, a hairdryer, an infrared ray dispenser or other standard heating appliances.

Use may be made, both as means for heating and straightening the head of hair, of a heating iron at a temperature ranging from 60°C to 220°C and preferably from 120°C to 200°C.

A particular form of the invention relates to a dyeing process which is performed at room temperature (25°C).

If, in the process, henna powder is the only ingredient a) as defined previously, it may be followed by another dyeing process as defined previously comprising, as ingredient a), indigo; and *vice versa*.

More particularly, the process of the invention is performed according to the following two processes:

- Process 1:

A composition **B** obtained from a composition **A** containing henna and/or indigo-producing plant(s) by mixing an amount of 1 part by weight and water at a temperature inclusively between 40 and 98°C for an amount of 2-3 parts by weight i) is applied to the head preferably in the form of a poultice for a minimum of 30 minutes, preferably between 30 minutes and 24 hours and better still between 1 hour and 12 hours, followed by ii) rinsing preferably without shampooing, and then iii) the hair is dried; next, optionally, iv) a heating iron at a temperature inclusively between 100 and 230°C and preferably between 130 and 200°C is used along the length of the head of hair;

- Process 2:

A composition **B** obtained from a composition **A** containing henna and/or indigo-producing plant(s) by mixing an amount of 1 part by weight and water at a temperature inclusively between 40 and 98°C for an amount of 2-3 parts by weight i) is preferably prepared in the form of a poultice, ii) composition B is then left to stand for a minimum of 30 minutes, preferably between 30 minutes and 48 hours and better still between 6 hours and 24 hours, and then iii) composition B is applied to the head and left to stand for a minimum of 30 minutes, preferably between 30 minutes and 24 hours and better still between 1 hour and 12 hours, followed by ii) rinsing preferably without shampooing, and then iii) the hair is dried; next, optionally, iv) a heating iron at a temperature inclusively between 100 and 230°C and preferably between 130 and 200°C is used along the length of the head of hair;

- Process 3:

A composition **B** obtained from a composition **A** containing henna and/or indigo-producing plant(s) by mixing an amount of 1 part by weight and water at a temperature inclusively between 10 and 40°C for an amount of 2-3 parts by weight i) is applied to the head preferably in the form of a poultice for a minimum of 30 minutes, preferably between 30 minutes and 24 hours and better still between 1 hour and 12 hours, followed by ii) rinsing preferably without shampooing, and then iii) the hair is dried; next, optionally, iv) a heating iron between 100 and 230°C and preferably between 130 and 200°C is used along the length of the head of hair;

To obtain the poultice **B** from composition **A** in compact form, the said composition **A** (1 part) is preferably crumbled in a container, for example a bowl, to which is added hot water **C** at a temperature of between 40 and 98°C, and the whole is then mixed until a creamy poultice is obtained.

A particular mode of the invention relates to a dyeing process which is performed at room temperature (25°C).

According to a particularly advantageous embodiment of the invention, all the ingredients used in the process are of natural origin, and preferably of plant origin.

The evaluation of the coloration can be done visually or read on a spectrocolorimeter (such as Minolta CM3600d, illuminant D65, angle 10°, SCI values) for the L*, a*, b* colorimetric measurements. In this L*, a*, b* system, L* represents the intensity of the color, a* indicates the green/red color axis and b* indicates the blue/yellow color axis. The lower the value of L, the darker or more intense the color. The higher the value of a*, the redder the shade; the higher the value of b*, the yellower the shade. The variation in coloring between the colored locks of natural white hair (NW) which is untreated (control) and after treatment or coloration are defined by ΔE*, corresponding to the colour uptake on keratin fibers, according to the following equation:

$$\Delta E^* = \sqrt{(L^* - L_o^*)^2 + (a^* - a_o^*)^2 + (b^* - b_o^*)^2}$$

In this equation, L*, a* and b* represent the values measured after dyeing the natural hair comprising 90% of white hairs and $L_0$*, $a_0$* and $b_0$* represent the values measured for the untreated natural hair comprising 90% of white hairs.

The greater the value of ΔE, the greater the difference in color between the control locks and the dyed locks

and the greater colour uptake is.

On the other hand for evaluating the selectivity of the color between the root and tip of the keratin fiber, measurement can be done on permed or sensibilised white hair (PW) and natural white hair, wherein the variation in coloring between the colored locks PW and the colored natural white hair are defined by $\Delta E^*$, corresponding to the selectivity of the colour, is calculated according to the following equation:

$$\Delta E^* = \sqrt{(L^* - L_o^*)^2 + (a^* - a_o^*)^2 + (b^* - b_o^*)^2}$$

In this equation, L*, a* and b* represent the values measured after dyeing the natural hair comprising 90% of white hairs and $L_0^*$, $a_0^*$ and $b_0^*$ represent the values measured after dyeing the permed or sensibilised hair. The lowest $\Delta E^*$, the best homogeneity of the hair color.

If the light fastness is investigated, $\Delta E^*$ is also calculated for the $L_0^*$, $a_0^*$, $b_0^*$ and L*, a*, b* measured of the locks before and after exposure to the light, respectively.

Chromaticity in the CIE L*, a*, b* colorimetric system is calculated according to the following equation :

$$C^* = \sqrt{a^{*2} + b^{*2}}$$

The greater the value of C*, the greater the chromaticity is.

The examples that follow serve to illustrate the invention without, however, being limiting in nature.

## I) EXAMPLES OF DYEING

The percentages are indicated on a weight basis relative to 100 g of composition.

**Composition A**:

| Ingredient | Commercial name | Amount |
|---|---|---|
| Natural henna (*Lawsonia inermis*) powder | Natural henna powder | 75 g% |
| Refined coconut oil | Refined copra oil GV 24/26 | 15 g% |
| Sorbitol | Sorbitol | 10 g% |

**Composition B**

| Ingredient | Commercial name | Amount |
|---|---|---|
| Natural henna (*Lawsonia inermis*) powder | Natural henna powder | 75 g% |
| Refined coconut oil | Refined copra oil GV 24/26 | 15 g% |
| Saccharose | Sucrose | 10 g% |

1 part by weight of one of the two compositions is mixed with 2 parts by weight of hot water (at about 60°C) in a bowl.

The mixing is performed easily, and the compositions, even compact, break down rapidly in water. The poultice obtained is very creamy, and is easy to apply to the keratin fibres, totally impregnating the keratin fibres from the root to the end.

The poultice is applied to dry natural grey hair containing 90% white hairs, with a leave-on time of 60 minutes.

The hair is rinsed thoroughly.

The hair is dried.

A strong coppery aesthetic coloration is obtained, with good homogeneity from the root to the end or from one fibre to another. The hair is soft and smooth.

**Comparative essays 1** :

| Ingredient | Composition C | Composition D |
|---|---|---|
| Natural henna (*Lawsonia inermis*) powder | 79 g% | 79 g% |
| Olive oil (invention) | 20 g% | - |
| Castor oil (comparative) | - | 20 g% |
| Sorbitol | 1 g% | 1 g% |

The poultice is applied to dry natural grey hair containing 90% white hairs, (bath ratio 10 g of composition for 1 g of hair) with a leave-on time of 60 minutes at room temperature (27 °C).
The hair is rinsed thoroughly with water (37°C).
The hair is dried.

**Results on intensity of the color L***

The evaluation of the coloration is done visually and read on a spectrocolorimeter (spectra flash SF600X, illuminant D65, angle 10°, SCI values) for the L*, a*, b* colorimetric measurements. In this L*, a*, b* system, wherein L* represents the intensity of the color. The lower the value of L, the darker or more intense the color.

| Intensity of the color | L* |
|---|---|
| Composition C (invention) | 44,99 |
| Composition D (comparative) | 48,69 |

Hair treated with composition of the invention C is significantly more intensive in terms of coloration than the one treated with the comparative composition D.

**Comparative essays 2** :

| Ingredient | Composition E | Composition F |
|---|---|---|
| Natural henna (*Lawsonia inermis*) powder | 79 g% | 79 g% |
| Olive oil (invention) | 20 g% | - |
| Castor oil (comparative) | - | 20 g% |
| Sodium alginate | 1 g% | 1 g% |

The poultice is applied to dry natural grey hair containing 90% white hairs, (bath ratio 10 g of composition for 1 g of hair) with a leave-on time of 60 minutes at room temperature (27 °C).
The hair is rinsed thoroughly with water (37°C).
The hair is dried.

**Results on chromaticity of the color C***

The evaluation of the coloration is done visually and read on the same spectrocolorimeter (spectra flash SF600X, illuminant D65, angle 10°, SCI values). Chromaticity in the CIE L*, a*, b* colorimetric system is calculated according to the following equation :

$$C^* = \sqrt{a^{*2} + b^{*2}}$$

The greater the value of C*, the greater the chromaticity is.

| Chromaticity of the color: C* | a* | b* | C* |
|---|---|---|---|
| Composition E (invention) | 24,93 | 36,40 | 44,12 |
| Composition F (comparative) | 20,63 | 30,54 | 36,86 |

Hair treated with composition of the invention E is significantly more chromatic in terms of coloration than the one treated with the comparative composition F.

## Claims

1. Cosmetic composition **A** comprising:

   a) at least 10% by weight, relative to the weight of the composition, of henna and/or indigo-producing plant powder; and
   b) at least one oil selected from jojoba oil, babassu oil, sunflower oil, olive oil, coconut oil, Brazil nut oil, marula oil, corn oil, argan oil, soybean oil, marrow oil, grapeseed oil, linseed oil, sesame oil, hazelnut oil, apricot oil, macadamia oil, arara oil, coriander oil, almond oil, avocado oil, shea butter oil, rapeseed oil, borage oil, evening primrose oil, pomegranate oil, mango oil, palm oil, cottonseed oil and copra oil ;
   c) at least one, optionally reduced, saccharide ;

   it being understood that:

   - the b)/c) weight ratio is greater than 1; and
   - when the composition contains indigo-producing plant powder, the saccharide is other than glucose.

2. Composition **A** according to the preceding claim, in compact and/or anhydrous form, preferably the composition is compact and anhydrous.

3. Composition **A** according to Claim 1 or 2, in which ingredient a) in powder form consists of fine particles with a size of less than or equal to 500 μm; preferentially, the powder consists of fine particles with sizes inclusively between 50 and 300 μm and more particularly between 10 and 200 μm.

4. Composition **A** according to any one of the preceding claims, in which ingredient a) represents only red henna powder.

5. Composition **A** according to any one of Claims 1 to 3, in which ingredient a) represents only powder of indigo-producing plant(s).

6. Composition **A** according to any one of Claims 1 to 3, in which ingredient a) represents a mixture of henna powder, preferably of red henna, and of powder of indigo-producing plant(s).

7. Composition **A** according to any one of the preceding claims, in which ingredient a) is in an amount inclusively between 10% and 90% by weight, more particularly between 15% and 70%, or even between 20% and 60% by weight and more particularly between 25% and 50% by weight, relative to the total weight of composition **A**.

8. Composition **A** according to the preceding claim, in which the oil(s) and ingredient b), are chosen from the oil(s) are chosen from oils of plant origin such as avocado oil, olive oil, coconut oil, copra oil, argan oil and sunflower oil; preferentially, the oil(s), ingredient b), are chosen from copra oil and olive oil.

9. Composition **A** according to any one of the preceding claims, which comprises one or more butters preferably chosen from butters of natural origin, and particularly of plant origin, such as those chosen from shea butter, Karité Nilotica butter (*Butyrospermum parkii*), galam butter, (*Butyrospermum parkii*), Borneo butter or fat or tengkawang tallow

(*Shorea stenoptera*), shorea butter, illipé butter, madhuca butter or *Bassia madhuca longifolia* butter, mowrah butter (*Madhuca latifolia*), katiau butter (*Madhuca mottleyana*), phulwara butter (*M. butyracea*), mango butter (*Mangifera indica*), murumuru butter (*Astrocaryum murumuru*), kokum butter (*Garcinia indica*), ucuuba butter (*Virola sebifera*), tucuma butter, painya butter (*Kpangnan*) (*Pentadesma butyracea*), coffee butter (*Coffea arabica*), apricot butter (*Prunus armeniaca*), macadamia butter (*Macadamia ternifolia*), grapeseed butter (*Vitis vinifera*), avocado butter (*Persea gratissima*), olive butter (*Olea europaea*), sweet almond butter (*Prunus amygdalus dulcis*), cocoa butter (*Theobroma cacao*) and sunflower butter; preferentially, the butter(s) according to the invention are chosen from murumuru butter, ucuuba butter, shorea butter, illipé butter, shea butter and cupuacu butter, and even more preferentially from murumuru butter and ucuuba butter.

**10.** Composition **A** according to any one of the preceding claims, in which the composition comprises ingredient b) and optionally one or more butters as defined in the preceding claim, in an amount inclusively between 1% and 80% by weight, relative to the total weight of the composition, more particularly between 5% and 60% by weight, preferentially between 10% and 40% by weight and more preferentially between 15% and 30% by weight, relative to the total weight of the said composition.

**11.** Composition **A** according to any one of the preceding claims, in which ingredient c) is chosen from monosaccharides, preferably chosen from:

i) *"aldoses"* or polyhydroxyaldehydes, preferably comprising between 4 and 6 carbon atoms, such as erythrose or threose, ribose, arabinose, xylose or lyxose, allose, altrose, glucose, mannose, gulose, idose, galactose and talose;
ii) *"ketoses"* or polyhydroxy ketones preferably comprising between 4 and 6 carbon atoms, such as erythrulose, ribulose, or xylulose, psicose, fructose, sorbose and tagatose; and
iii) reduced forms of these aldoses and ketoses as defined previously, also known as *"sugar alcohols"* or *"alditols"*, in particular chosen from erythritol, glucitol, sorbitol, mannitol and xylitol, preferably sorbitol; and also the optical isomers thereof, the $\alpha$ and $\beta$ anomers thereof, and the L and D forms thereof.

**12.** Composition **A** according to any one of the preceding claims, in which ingredient c) is chosen from: oligosaccharides preferably chosen from disaccharides such as sucrose or saccharose, trehalose and raffinose, lactose, cellobiose and maltose; $\alpha$, $\beta$ or $\gamma$-cyclodextrins, and the "sugar alcohol" reduced forms thereof such as isomaltulose, trehalulose, isomalt, maltitol and lactitol, and also the optical isomers thereof, the $\alpha$ and $\beta$ anomers thereof and the L and D forms thereof.

**13.** Composition **A** according to any one of Claims 1 to 10, in which ingredient c) is chosen from polysaccharides comprising between 20 and 100 000 monosaccharide units preferably chosen from celluloses, guar gums, xanthan gums, chitins and chitosans, starches including amylose and amylopectin, dextrans and inulins.

**14.** Composition **A** according to any one of the preceding claims, in which ingredient c) is chosen from aldoses, ketoses, and the reduced forms thereof, namely sugar alcohols such as sorbitol.

**15.** Composition **A** according to any one of the preceding claims, in which the saccharide(s) or the reduced forms thereof, ingredient c), are in an amount inclusively between 0.2% and 50% by weight, relative to the total weight of composition **A**, more particularly between 2% and 30% by weight, preferentially between 3% and 20% by weight and more preferentially between 5% and 15% by weight relative to the total weight of the said composition.

**16.** Aqueous composition **B** prepared from a mixture of a composition **A** according to any one of the preceding claims and an aqueous composition **C** and preferably water, in proportions ranging from 1 part by weight of composition according to any one of the preceding claims per 1 part by weight of an aqueous composition **C** and preferably water (1/1) to 1 part by weight of composition according to any one of the preceding claims per 3 parts by weight of an aqueous composition **C** and preferably water (1/3), more preferentially 1 part by weight of composition according to any one of the preceding claims per 2 parts by weight of an aqueous composition **C** and preferably water by weight (1/2); composition **B** preferably being in the form of a poultice.

**17.** Composition **B** according to the preceding claim, containing water in an amount ranging from 5% to 90%, better still from 20% to 80% and particularly from 40% to 75% by weight relative to the total weight of the said composition.

**18.** Composition **A** or **B** according to any one of the preceding claims, in which the composition comprises one or more

surfactants.

**19.** Composition **A** or **B** according to any one of the preceding claims, in which the composition consists only of ingredients of natural origin.

**20.** Composition **B** according to Claims 17 to 19, which is at a pH ranging from 6 to 8 and better still from 6.5 to 7.5.

**21.** Process for dyeing keratin fibres, in particular human keratin fibres such as the hair, involving the following steps:

- in the first step, the preparation of a composition **B** according to Claims 16 to 20;
- in the second step, composition **B** is:

  a) either immediately applied to the keratin fibres, and left on the fibres preferably for a minimum time of 30 minutes, preferentially ranging from 30 minutes to 24 hours and in particular from 1 hour to 12 hours,
  b) or left to stand for several hours, preferably 24 hours, and then applied and left on the fibres preferably for a minimum time of 30 minutes, preferably ranging from 30 minutes to 24 hours and in particular from 1 hour to 12 hours;

- in the third step, the keratin fibres are rinsed with water until the poultice has disappeared, preferably without shampooing;
- the keratin fibres may then be dried with a source of heat or left to dry naturally at room temperature.

**22.** Use of composition **A** according to any one of Claims 1 to 15, 18 or 19 or of composition **B** according to any one of Claims 16 to 20, for dyeing keratin fibres such as the hair.

**Patentansprüche**

**1.** Kosmetische Zusammensetzung A, umfassend:

a) mindestens 10 Gewichts%, bezogen auf das Gewicht der Zusammensetzung, Henna und/oder eines Indigo erzeugenden Pflanzenpulvers; und
b) mindestens ein Öl, ausgewählt aus Jojobaöl, Babassuöl, Sonnenblumenöl, Olivenöl, Kokosnussöl, Paranussöl, Marulaöl, Maisöl, Arganöl, Sojabohnenöl, Kürbisöl, Traubenkernöl, Leinöl, Sesamöl, Haselnussöl, Aprikosenöl, Macadamiaöl, Araraöl, Korianderöl, Mandelöl, Avocadoöl, Sheabutteröl, Rapsöl, Borretschöl, Nachtkerzenöl, Granatapfelöl, Mangoöl, Palmöl, Baumwollsamenöl und Kokosöl;
c) mindestens ein, optional reduziertes, Saccharid;

wobei es sich versteht, dass

- das b)/c)-Gewichtsverhältnis größer ist als 1; und
- wenn die Zusammensetzung Indigo erzeugendes Pflanzenpulver enthält, das Saccharid keine Glucose ist.

**2.** Zusammensetzung A nach dem vorhergehenden Anspruch in kompakter und/oder wasserfreier Form, wobei die Zusammensetzung vorzugsweise kompakt und wasserfrei ist.

**3.** Zusammensetzung A nach Anspruch 1 oder 2, wobei der Bestandteil a) in Pulverform aus feinen Partikeln mit einer Größe von kleiner als oder gleich 500 μm besteht; wobei das Pulver bevorzugt aus feinen Partikeln mit Größen einschließlich zwischen 50 und 300 μm und insbesondere zwischen 10 und 200 μm besteht.

**4.** Zusammensetzung A nach einem der vorhergehenden Ansprüche, wobei der Bestandteil a) nur rotes Hennapulver aufweist.

**5.** Zusammensetzung A nach einem der Ansprüche 1 bis 3, wobei der Bestandteil a) nur Pulver (einer) Indigo erzeugenden/r Pflanze(n) aufweist.

**6.** Zusammensetzung A nach einem der Ansprüche 1 bis 3, wobei der Bestandteil a) ein Gemisch von Hennapulver, vorzugsweise rotem Henna, und von Pulver (einer) Indigo erzeugenden/r Pflanze(n) aufweist.

**7.** Zusammensetzung A nach einem der vorhergehenden Ansprüche, wobei der Bestandteil a) in einer Menge einschließlich zwischen 10 und 90 Gewichts%, insbesondere zwischen 15 und 70 Gewichts% oder sogar zwischen 20 und 60 Gewichts% und insbesondere zwischen 25 und 50 Gewichts%, bezogen auf das Gesamtgewicht der Zusammensetzung A, vorliegt.

**8.** Zusammensetzung A nach dem vorhergehenden Anspruch, wobei das/die Öl(e) und Bestandteil b), aus dem/n Öl(en) ausgewählt sind aus Ölen pflanzlichen Ursprungs ausgewählt sind, wie etwa Avocadoöl, Olivenöl, Kokosnussöl, Kokosöl, Arganöl und Sonnenblumenöl; wobei das/die Öl(e), Bestandteil b), bevorzugt aus Kokosöl und Olivenöl ausgewählt sind.

**9.** Zusammensetzung A nach einem der vorhergehenden Ansprüche, die eine oder mehrere Butter (n) umfasst, vorzugsweise ausgewählt aus Buttern natürlichen Ursprungs und insbesondere pflanzlichen Ursprungs, wie etwa denjenigen, ausgewählt aus Sheabutter, Karite-Nilotica-Butter (*Butyrospermum parkii*), Galambutter *(Butyrospermum parkii*), Borneobutter oder -fett oder Tengkawang-Talg (*Shorea stenoptera*), Shoreabutter, Illipé-Butter, Madhuca-Butter oder *Bassia-madhucalongifolia*-Butter, Mowrah-Butter (*Madhuca latifolia*), Katiau-Butter (*Madhuca mottleyana*), Phulwara-Butter (*M. butyracea*), Mangobutter (*Mangifera indica*), Murumuru-Butter (*Astrocaryum murumuru*), Kokum-Butter (*Garcinia indica*), Ucuuba-Butter (*Virola sebifera*), Tucuma-Butter, Painya-Butter (*Kpangnan*) (*Pentadesma butyracea*), Kaffeebutter (*Coffea arabica*), Aprikosenbutter (*Prunus armeniaca*), Macadamiabutter (*Macadamia ternifolia*), Traubenkernbutter (*Vitis vinifera*), Avocadobutter (*Persea gratissima*), Olivenbutter (*Olea europaea*), Süßmandelbutter (*Prunus amygdalus dulcis*), Kakaobutter (*Theobroma cacao*) und Sonnenblumenbutter; wobei die Butter(n) gemäß der Erfindung bevorzugt aus Murumuru-Butter, Ucuuba-Butter, Shoreabutter,Illipé-Butter, Sheabutter und Cupuacu-Butter und besonders bevorzugt aus Murumuru-Butter und Ucuuba-Butter ausgewählt sind.

**10.** Zusammensetzung A nach einem der vorhergehenden Ansprüche, wobei die Zusammensetzung den Bestandteil b) und optional eine oder mehrere Buttern, wie im vorhergehenden Anspruch definiert, in einer Menge einschließlich zwischen 1 und 80 Gewichts%, bezogen auf das Gesamtgewicht der Zusammensetzung, insbesondere zwischen 5 und 60 Gewichts%, bevorzugt zwischen 10 und 40 Gewichts% und besonders bevorzugt zwischen 15 und 30 Gewichts%, bezogen auf das Gesamtgewicht der Zusammensetzung, umfasst.

**11.** Zusammensetzung A nach einem der vorhergehenden Ansprüche, wobei der Bestandteil c) aus Monosacchariden ausgewählt ist, vorzugsweise ausgewählt aus:

i) *"Aldosen"* oder Polyhydroxyaldehyden, die vorzugsweise zwischen 4 und 6 Kohlenstoffatome umfassen, wie etwa Erythrose oder Threose, Ribose, Arabinose, Xylose oder Lyxose, Allose, Altrose, Glucose, Mannose, Gulose, Idose, Galactose und Talose;
ii) *"Ketosen"* oder Polyhydroxyketonen, die vorzugsweise zwischen 4 und 6 Kohlenstoffatome umfassen, wie etwa Erythrulose, Ribulose oder Xylulose, Psicose, Fructose, Sorbose und Tagatose; und
iii) reduzierten Formen dieser Aldosen und Ketosen, wie zuvor definiert, auch *"zuckeralkohole"* oder *"Alditole"* genannt, insbesondere ausgewählt aus Erythritol, Glucitol, Sorbitol, Mannitol und Xylitol, vorzugsweise Sorbitol; und auch den optischen Isomeren derselben, den $\alpha$- und $\beta$-Anomeren derselben und den L- und D-Formen derselben.

**12.** Zusammensetzung A nach einem der vorhergehenden Ansprüche, wobei der Bestandteil c) ausgewählt ist aus:

Oligosacchariden, vorzugsweise ausgewählt aus Disacchariden, wie etwa Sucrose oder Saccharose, Trehalose und Raffinose, Lactose, Cellobiose und Maltose; $\alpha$-, $\beta$- oder $\gamma$-Cyclodextrinen und den reduzierten "Zuckeralkohol"-Formen derselben, wie etwa Isomaltulose, Trehalulose, Isomalt, Maltitol und Lactitol, und auch den optischen Isomeren derselben, den $\alpha$- und $\beta$-Anomeren derselben und den L- und D-Formen derselben.

**13.** Zusammensetzung A nach einem der Ansprüche 1 bis 10, wobei der Bestandteil c) aus Polysacchariden ausgewählt ist, die zwischen 20 und 100.000 Monosaccharideinheiten umfassen, vorzugsweise ausgewählt aus Cellulosen, Guargummis, Xanthangummis, Chitinen und Chitosanen, Stärken einschließlich Amylose und Amylopektin, Dextranen und Inulinen.

**14.** Zusammensetzung A nach einem der vorhergehenden Ansprüche, wobei der Bestandteil c) aus Aldosen, Ketosen und den reduzierten Formen derselben, also Zuckeralkoholen, wie etwa Sörbitol, ausgewählt ist.

**15.** Zusammensetzung A nach einem der vorhergehenden Ansprüche, wobei das/die Saccharid(e) oder die reduzierten

Formen des/derselben, Bestandteil c), in einer Menge einschließlich zwischen 0,2 und 50 Gewichts%, bezogen auf das Gesamtgewicht der Zusammensetzung A, insbesondere zwischen 2 und 30 Gewichts%, bevorzugt zwischen 3 und 20 Gewichts% und besonders bevorzugt zwischen 5 und 15 Gewichts%, bezogen auf das Gesamtgewicht der Zusammensetzung, vorliegen.

**16.** Wässrige Zusammensetzung B, hergestellt aus einem Gemisch einer Zusammensetzung A nach einem der vorhergehenden Ansprüche und einer wässrigen Zusammensetzung C und vorzugsweise Wasser, in Anteilen im Bereich von 1 Gewichtsteil der Zusammensetzung nach einem der vorhergehenden Ansprüche pro 1 Gewichtsteil einer wässrigen Zusammensetzung C und vorzugsweise Wasser (1/1) bis 1 Gewichtsteil der Zusammensetzung nach einem der vorhergehenden Ansprüche pro 3 Gewichtsteile einer wässrigen Zusammensetzung C und vorzugsweise Wasser (1/3), besonders bevorzugt von 1 Gewichtsteil der Zusammensetzung nach einem der vorhergehenden Ansprüche pro 2 Gewichtsteile einer wässrigen Zusammensetzung C und vorzugsweise Wasser nach Gewicht (1/2); wobei die Zusammensetzung B vorzugsweise in Form eines Breis vorliegt.

**17.** Zusammensetzung B nach dem vorhergehenden Anspruch, die Wasser in einer Menge im Bereich von 5 bis 90 Gewichts%, noch besser von 20 bis 80 Gewichts% und insbesondere von 40 bis 75 Gewichts%, bezogen auf das Gesamtgewicht der Zusammensetzung, enthält.

**18.** Zusammensetzung A oder B nach einem der vorhergehenden Ansprüche, wobei die Zusammensetzung ein oder mehrere oberflächenaktive(s) Mittel umfasst.

**19.** Zusammensetzung A oder B nach einem der vorhergehenden Ansprüche, wobei die Zusammensetzung nur aus Bestandteilen natürlichen Ursprungs besteht.

**20.** Zusammensetzung B nach den Ansprüchen 17 bis 19, die einen pH-Wert im Bereich von 6 bis 8 und noch besser von 6,5 bis 7,5 aufweist.

**21.** Verfahren zum Färben von Keratinfasern, insbesondere menschlichen Keratinfasern, wie etwa dem Haar, das folgende Schritte beinhaltet:

- im ersten Schritt, die Herstellung einer Zusammensetzung B nach den Ansprüchen 16 bis 20;
- im zweiten Schritt wird die Zusammensetzung B

  a) entweder unverzüglich auf die Keratinfasern aufgebracht und vorzugsweise für eine Mindestzeit von 30 Minuten, bevorzugt im Bereich von 30 Minuten bis 24 Stunden und insbesondere von 1 Stunde bis 12 Stunden, auf den Fasern belassen,
  b) oder mehrere Stunden, vorzugsweise 24 Stunden, stehen gelassen und dann auf die Fasern aufgebracht und vorzugsweise für eine Mindestzeit von 30 Minuten, vorzugsweise im Bereich von 30 Minuten bis 24 Stunden und insbesondere von 1 Stunde bis 12 Stunden, auf diesen belassen;

- im dritten Schritt werden die Keratinfasern mit Wasser gespült, bis der Brei verschwunden ist, vorzugsweise ohne Shampoonieren;
- die Keratinfasern können dann mit einer Wärmequelle getrocknet werden oder natürlich bei Raumtemperatur trocknen.

**22.** Verwendung der Zusammensetzung A nach einem der Ansprüche 1 bis 15, 18 oder 19 oder der Zusammensetzung B nach einem der Ansprüche 16 bis 20 zum Färben von Keratinfasern, wie etwa dem Haar.

## Revendications

**1.** Composition cosmétique **A** comprenant :

a) au moins 10 % en poids, par rapport au poids de la composition, de poudre de henné, et/ou de plante indigofère; et
b) au moins une huile choisie parmi les huiles de jojoba, de babassu, de tournesol, d'olive, de noix de coco, de noix du Brésil, de marula, de maïs, d'argan, de soja, de courge, de pépins de raisin, de lin, de sésame, de noisette, d'abricot, de macadamia, d'arara, de coriandre, d'amande, d'avocat, de beurre de karité, de colza, de

bourrache, d'onagre, de grenade, de mangue, de palme, de graine de coton et de coprah,
c) au moins un saccharide éventuellement réduit ;

entendu que :

- le rapport en poids b)/c) est supérieur à 1 ; et
- lorsque la composition contient de la poudre de plante indigofère, le saccharide est différent du glucose.

2. Composition **A** selon la revendication précédente, sous forme compacte et/ou anhydre, de préférence la composition est compacte et anhydre.

3. Composition **A** selon la revendication 1 ou 2, dans laquelle l'ingrédient a) sous forme de poudre est constitué de fines particules de taille inférieure ou égale à 500 μm ; préférentiellement la poudre est constituée de fines particules de taille comprise inclusivement entre 50 et 300 μm et plus particulièrement entre 10 et 200 μm.

4. Composition **A** selon une quelconque des revendications précédentes, dans laquelle l'ingrédient a) représente uniquement de la poudre de henné rouge.

5. Composition **A** selon une quelconque des revendications 1 à 3, dans laquelle l'ingrédient a) représente uniquement de la poudre de plante (s) indigofère(s).

6. Composition **A** selon une quelconque des revendications 1 à 3, dans laquelle l'ingrédient a) représente un mélange de poudre de henné, de préférence de henné rouge, et de poudre de plante(s) indigofère(s).

7. Composition **A** selon une quelconque des revendications précédentes, dans laquelle l'ingrédient a) se trouve en quantité comprise inclusivement entre 10 % et 90 % en poids, plus particulièrement entre 15 et 70 %, voire entre 20 et 60 % en poids, plus particulièrement entre 25 et 50 % en poids, par rapport au poids total de la composition **A**.

8. Composition **A** selon la revendication précédente, dans laquelle l'huile ou les huiles et l'ingrédient b) sont choisis parmi l'huile ou les huiles choisis parmi les huiles d'origine végétale telles que l'huile d'avocat, l'huile d'olive, l'huile de noix coco, l'huile de coprah, l'huile d'argan et l'huile de tournesol ; plus préférentiellement l'huile ou les huiles, l'ingrédient b), sont choisis parmi l'huile de coprah et l'huile d'olive.

9. Composition **A** selon une quelconque des revendications précédentes, qui comprend un ou plusieurs beurres, de préférence choisis parmi les beurres d'origine naturelle, et particulièrement d'origine végétale tels que ceux choisis parmi le beurre de karité, le beurre de Karité Nilotica (*Butyrospermum parkii*), le beurre de Galam, (*Butyrospermum parkii*), le beurre ou graisse de Bornéo ou tengkawang tallow (*Shorea stenoptera*), le beurre de Shorea, beurre d'Illipé, le beurre de Madhuca ou *Bassia Madhuca longifolia,* le beurre de mowrah (*Madhuca Latifolia*), le beurre de Katiau (*Madhuca mottleyana*), le beurre de Phulwara (*M. butyracea*), le beurre de mangue (*Mangifera indica*), le beurre de Murumuru (*Astrocaryum murumuru*), le beurre de Kokum (*Garcinia Indica*), le beurre d'Ucuuba (*Virola sebifera*), le beurre de Tucuma, le beurre de Painya (*Kpangnan*) (*Pentadesma butyracea*), le beurre de café (*Coffea arabica*), le beurre d'abricot (*Prunus Armeniaca*), le beurre de Macadamia (*Macadamia Ternifolia*), le beurre de pépin de raisin (*Vitis vinifera*), le beurre d'avocat (*Persea gratissima*), le beurre d'olives (*Olea europaea*), le beurre d'amande douce (*Prunus amygdalus dulcis*), le beurre de cacao (*Theobroma cacao*) et le beurre de tournesol ; préférentiellement le ou les beurres selon l'invention sont choisis parmi le beurre de Murumuru, le beurre d'Ucuuba, le beurre de Shorea, le beurre d'Illipé, le beurre de Karité et le beurre de Cupuaçu et encore plus préférentiellement parmi le beurre de Murumuru et le beurre d'Ucuuba.

10. Composition **A** selon une quelconque des revendications précédentes, dans laquelle la composition comprend l'ingrédient b) et éventuellement un ou plusieurs beurres tels que définis dans la revendication précédente, en quantité comprise inclusivement entre 1 et 80 % en poids par rapport au poids total de la composition, plus particulièrement comprise entre 5 et 60 % en poids, préférentiellement comprise entre 10 et 40 % en poids, et plus préférentiellement comprise entre 15 et 30 % en poids par rapport au poids total de ladite composition.

11. Composition **A** selon une quelconque des revendications précédentes, dans laquelle l'ingrédient c) est choisi parmi les monosaccharides, de préférence choisis parmi :

i) les « *aLdoses* » ou polyhydroxyaldéhydes, comprenant de préférence entre 4 et 6 atomes de carbone tels

que l'érythose ou le thréose, le ribose, l'arabinose, le xylose ou le lyxose, l'allose, l'altrose, le glucose, le mannose, le gulose, l'idose, le galactose et le talose ;
ii) les « *cétoses* » ou polyhydroxycétones comprenant de préférence entre 4 et 6 atomes de carbone tels que l'érythrulose, le ribulose ou le xylulose, le psicose, le fructose, le sorbose et le tagatose ; et
iii) les formes réduites de ces aldoses et cétoses tels que définis précédemment également appelés « *sucre alcools* » ou « *alditols* », en particulier choisi parmi l'érythritol, le glucitol, le sorbitol, le mannitol et le xylitol, de préférence le sorbitol ; ainsi que leurs isomères optiques, leurs anomères $\alpha$ et $\beta$ et leurs formes L et D.

**12.** Composition **A** selon une quelconque des revendications précédentes, dans laquelle l'ingrédient c) est choisi parmi : les oligosaccharides de préférence choisis parmi les disaccharides tels que le sucrose ou saccharose, le tréhalose et raffinose, le lactose, cellobiose et maltose ; les $\alpha$, $\beta$ ou $\gamma$ cyclodextrines, et leurs formes réduites « sucres alcool » tels que l'isomaltulose, le tréhalulose, l'isomalt, le maltitol, et le lactitol, ainsi que leurs isomères optiques, leurs anomères $\alpha$ et $\beta$ et leurs formes L et D .

**13.** Composition **A** selon une quelconque des revendications 1 à 10, dans laquelle l'ingrédient c) est choisi parmi les polysaccharides comportant entre 20 et 100 000 unités monosaccharides de préférence choisis parmi les celluloses, les gommes de guar et de xanthane, les chitines et chitosans, les amidons dont l'amylose, l'amylopectine, les dextranes, et les inulines.

**14.** Composition **A** selon une quelconque des revendications précédentes, dans laquelle l'ingrédient c) est choisi parmi les aldoses, les cétoses, et leurs formes réduites que sont les sucres alcools tels que le sorbitol.

**15.** Composition **A** selon une quelconque des revendications précédentes, dans laquelle le ou les saccharides ou leurs formes réduites, l'ingrédient c), se trouvent en quantité comprise inclusivement entre 0,2 et 50 % en poids par rapport au poids total de la composition **A**, plus particulièrement comprise entre 2 et 30 % en poids, préférentiellement comprise entre 3 et 20 % en poids, et plus préférentiellement comprise entre 5 et 15 % en poids par rapport au poids total de ladite composition.

**16.** Composition aqueuse **B** réalisée à partir de mélange d'une composition **A** selon une quelconque des revendications précédentes, et d'une composition aqueuse **C** et de préférence d'eau, dans des proportions allant de 1 part en poids de composition selon l'une quelconque des revendications précédentes pour 1 part en poids d'une composition aqueuse **C** et de préférence d'eau (1/1) à 1 part en poids de composition selon l'une quelconque des revendications précédentes pour 3 parts en poids d'une composition aqueuse **C** et de préférence d'eau (1/3), plus préférentiellement 1 part en poids de composition selon l'une quelconque des revendications précédentes pour 2 parts en poids d'une composition aqueuse **C** et de préférence d'eau en poids (1/2) ; la composition **B** se présentant de préférence sous forme d'un cataplasme.

**17.** Composition **B** selon la revendication précédente, contenant de l'eau en une quantité allant de 5 à 90 %, mieux de 20 à 80 % et particulièrement de 40 à 75 % en poids par rapport au poids total de ladite composition.

**18.** Composition **A** ou **B** selon une quelconque des revendications précédentes, dans laquelle la composition comprend un ou plusieurs tensioactifs.

**19.** Composition **A** ou **B** selon une quelconque des revendications précédentes, dans laquelle la composition n'est constituée que d'ingrédients d'origine naturelle.

**20.** Composition **B** selon les revendications 17 à 19, qui se trouve à un pH allant de 6 à 8, mieux de 6,5 à 7,5.

**21.** Procédé de coloration de fibres kératiniques, en particulier de fibres kératiniques humaines telles que les cheveux, mettant en oeuvre les étapes suivantes :

- lors de la première étape, la préparation d'une composition **B** selon les revendications 16 à 20 ;
- lors de la deuxième étape la composition **B** est :

a) soit immédiatement appliquée sur les fibres kératiniques, et est laissée sur les fibres de préférence un temps minimum de 30 minutes, préférentiellement allant de 30 minutes à 24 heures, en particulier de 1 heure à 12 heures,
b) soit laissée reposer pendant plusieurs heures, de préférence 24 heures, puis appliquée et laissée sur

les fibres de préférence un temps minimum de 30 minutes, de préférence allant de 30 minutes à 24 heures, en particulier de1 heure à 12 heures ;

- lors de la troisième étape, les fibres kératiniques sont rincées à l'eau jusqu'à disparition du cataplasme, de préférence sans faire de shampoing ;
- les fibres kératiniques peuvent ensuite être séchées avec une source de chaleur ou laissées sécher naturellement à température ambiante.

**22.** Utilisation de la composition **A** selon une quelconque des revendications 1 à 15, 18 ou 19, ou de la composition **B** selon une quelconque des revendications 16 à 20, pour colorer les fibres kératiniques telles que les cheveux.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 201003133362 A
- EP 1631241 A
- DE 19905707
- EP 0806199 A
- JP 2010001278 A
- US 3589578 A
- US 40131307 B
- US 5455340 A
- US 4017460 A


### Non-patent literature cited in the description


- Ullmann's Encyclopedia of Industrial Chemistry, Hair preparation, point 5.2.3. Wiley-VCH Verlag GmbH & Co. KGaA, 2006
- *Kirk-Othmer Encyclopedia of Chemical Technology,* 17 April 2009
- *Chem. Rev.,* 2011, vol. 111, 2537-2561
- Hair preparations. Kirk-Othmer Encyclopedia of Chemical Technology. John Wiley & Sons, Inc,
- **C. BOUILLON ; J. WILKINSON.** The Science of Hair Care. Taylor & Francis Group, 2005, 236-241
- Ullmann's Encyclopedia. Wiley-VCH Verlag GmbH & Co. KGaA, 2006
- Fats and Fatty Oils. **A. THOMAS.** Ullmann's Encyclopedia of Industrial Chemistry. 15 June 2000
- Carbohydrates: Occurrence, Structures and Chemistry. **F. W. LICHTENTHALER.** Ullmann's Encyclopedia of Industrial Chemistry. 15 April 2010, vol. 6, 617-646
- Carbohydrates: Occurrence, Structures and Chemistry. **F. W. LICHTENTHALER.** Ullmann's Encyclopedia of Industrial Chemistry. 15 April 2010, vol. 6, 637, , 638
- **H. SCHIWECK et al.** Sugar Alcohols. Wiley-VCH Verlag GmbH & Co. KGaA, 15 January 2012
- Textile Dyeing. **HERBERT LEUBE et al.** Ullmann's Encyclopedia of Industrial Chemistry
- **KLAUS GRYSCHTOL et al.** *Metal-complex dyes*